# EUROPEAN PATENT APPLICATION

(11) **EP 4 810 056 A2**
(43) Date of publication of application: **23.09.2026**
(21) Application number: 26191156.4
(22) Date of filing: 16.05.2024
(51) Int. Cl.: A61K 39/00

(54) **METHODS AND COMBINATIONS FOR TREATING CERVICAL CANCER**

(30) Priority: 17.05.2023 US 202363502740 P
(62) Document number(s) of the earlier application(s) in accordance with Art. 76 EPC: 24729091.9 / 4 713 362
(71) Applicant: Astrazeneca AB, 151 85 Södertälje (SE)
(72) Inventor: KIM, Geoffrey, Gaithersburg, Maryland 20878 (US); LANASA, Mark, Gaithersburg, Maryland 20878 (US); NUNES, Ana, Gaithersburg, Maryland 20878 (US); SUBRAMANIAM, Deepa, Gaithersburg, Maryland 20878 (US); WILDSMITH, Sophie, Cambridge, Cambridgeshire (GB)
(74) Representative: AstraZeneca Intellectual Property

(57) **Abstract**

The disclosure relates to methods, combinations, and uses for treating cervical cancer using an anti-PD-L1 binding protein or an anti-PD-1 binding protein and chemoradiation therapy.

## Description

### REFERENCE TO SEQUENCE LISTING

This application contains a sequence listing which is submitted electronically and is hereby incorporated by reference in its entirety. The sequence listing submitted herewith is contained in the XML filed created May 3, 2024 entitled "B7H1-280-US-PSP_Sequence-Listing.xml" and is 15,863 bytes in size.

### FIELD OF THE DISCLOSURE

The disclosure relates to methods, combinations, and uses for treating cervical cancer using an anti-PD-1 binding protein or an anti-PD-L1 binding protein and chemoradiation therapy.

### BACKGROUND

Cervical cancer is the 4th most common cancer in women worldwide despite the introduction of the Pap smear for early screening and prevention of cervical cancer in the 1950s, and more recently, HPV vaccination. Many women continue to be diagnosed with and die from this disease, with an estimated 528,000 new cases diagnosed in 2012 and 266,000 deaths (Ferlay et al., GLOBOCAN 2012 v1.0, Cancer incidence and mortality worldwide: IARC CancerBase No. 11 [serial online] 2013). North America has the third lowest rate of cervical cancer. However, in the U.S. there were 12,109 women diagnosed with cervical cancer and 4,092 deaths in 2011 (Benard et al., "Vital signs: cervical cancer incidence, mortality, and screening-United States, 2007-2012," MMWR Morb. Mortal Wkly. Rep. 63(44): 1004-09 (2014)). In contrast, in China, 98,900 women were diagnosed with cervical cancer and 30,500 women died of the disease in 2015 (Chen et al., "Cancer statistics in China, 2015," CA Cancer J. Clin. 66(2): 115-32 (2016)). Overall, the 5-year overall survival in this patient population remains poor with survival at Stage IIB 58%, IIIA 35%, and IVA 16% (Ferlay et al., GLOBOCAN 2012 v1.0, Cancer incidence and mortality worldwide: IARC CancerBase No. 11 [serial online] 2013). Rates of cervical cancer vary greatly between the developing world and developed countries due to disparities in access to care and preventive screening.

In addressing the need for improved methods and combinations for clinical management of locally advanced cancers, the disclosure provides methods, combinations, and uses for the treatment of patients with Locally Advanced Cervical Cancer (LACC) using an anti-PD-1 binding protein or an anti-PD-L1 binding protein, with or without a chemoradiation therapy.

### SUMMARY

The disclosure relates to methods, combinations, and uses for treating patients who have cervical cancer, comprising administering to the patient an anti-PD-1 binding protein or an anti-PD-L1 binding protein, and in some aspects, a chemoradiation therapy. The disclosed methods, combinations, and uses for treatment can provide for substantial improvement in a patient's progression-free survival (PFS), overall response rate (ORR), and/or overall survival (OS).

In an aspect, this disclosure provides a method of treating cervical cancer, the method comprising treating the patient with an anti-PD-1 binding protein or an anti-PD-L1 binding protein. In some aspects, the method further comprises treating the patient with a chemoradiation therapy. In some aspects, the cervical cancer is locally advanced cervical cancer (LACC). In some aspects, the patient has a tumor area positivity (TAP) score of ≥ 20% for PD-L1 expression.

In some aspects, the anti-PD-1 binding protein or the anti-PD-L1 binding protein is an anti-PD-1 antibody selected from pembrolizumab, nivolumab, and cemiplimab. In some aspects, the anti-PD-1 binding protein or the anti-PD-L1 binding protein is an anti-PD-L1 antibody selected from durvalumab, avelumab, atezolizumab, and sugemalimab. In some aspects, the anti-PD-1 binding protein or the anti-PD-L1 binding protein is a bispecific antibody, wherein the bispecific antibody is MEDI5752.

In some aspects, the chemoradiation therapy comprises concurrent chemoradiation therapy. In some aspects, the chemoradiation therapy comprises external beam radiotherapy and brachytherapy. In some aspects, the chemoradiation therapy comprises a platinum-based chemotherapy. In some aspects, the platinum-based chemotherapy is cisplatin or carboplatin.

In some aspects, treatment with the anti-PD-L1 antibody comprises administering 1500 mg of the anti-PD-L1 antibody to the patient intravenously every four weeks (Q4W). In some aspects, treatment with the anti-PD-L1 antibody comprises administering 1500 mg of the anti-PD-L1 antibody to the patient intravenously every three weeks (Q3W). In some aspects, the anti-PD-L1 antibody is durvalumab.

In some aspects, treatment with the bispecific antibody comprises administering 750 mg of the bispecific antibody to the patient intravenously every three weeks (Q3W). In some aspects, treatment with the bispecific antibody comprises administering 500 mg or 750 mg of MEDI5752 to the patient intravenously every three weeks (Q3W).

In some aspects, the anti-PD-1 binding protein or the anti-PD-L1 binding protein, and the chemoradiation therapy are administered concurrently. In some aspects, the anti-PD-1 binding protein or the anti-PD-L1 binding protein is administered after the chemoradiation therapy.

In some aspects, the method extends progression free survival (PFS) in the patient. In some aspects, the method increases the overall response rate (ORR) in the patient.

In an aspect, this disclosure provides a combination for use in the treatment of cervical cancer in a patient, wherein the combination comprises an anti-PD-1 binding protein or an anti-PD-L1 binding protein. In some aspects, the combination further comprises a chemoradiation therapy. In some aspects, the cervical cancer is locally advanced cervical cancer (LACC). In some aspects, the patient has a tumor area positivity (TAP) score of ≥ 20% for PD-L1 expression.

In some aspects of the combination for use, the anti-PD-1 binding protein or the anti-PD-L1 binding protein is an anti-PD-1 antibody selected from pembrolizumab, nivolumab, and cemiplimab. In some aspects, the anti-PD-1 binding protein or the anti-PD-L1 binding protein is an anti-PD-L1 antibody selected from durvalumab, avelumab, atezolizumab, and sugemalimab. In some aspects, the anti-PD-1 binding protein is a bispecific antibody, wherein the bispecific antibody is MEDI5752.

In some aspects of the combination for use, the chemoradiation therapy comprises concurrent chemoradiation therapy. In some aspects, the chemoradiation therapy comprises external beam radiotherapy and brachytherapy. In some aspects, the chemoradiation therapy comprises a platinum-based chemotherapy. In some aspects, the platinum-based chemotherapy is cisplatin or carboplatin.

In some aspects of the combination for use, the combination comprises administering 1500 mg of the anti-PD-L1 antibody to the patient intravenously every four weeks (Q4W). In some aspects of the combination for use, the combination comprises administering 1500 mg of the anti-PD-L1 antibody to the patient intravenously every three weeks (Q3W). In some aspects of the combination for use, the anti-PD-L1 antibody is durvalumab.

In some aspects of the combination for use, the combination treatment comprises administering 500 mg or 750 mg of the bispecific antibody to the patient intravenously every three weeks (Q3W). In some aspects of the combination for use, the combination treatment comprises administering 500 mg or 750 mg of MEDI5752 to the patient intravenously every three weeks (Q3W).

In some aspects of the combination for use, the anti-PD-1 binding protein or the anti-PD-L1 binding protein, and the chemoradiation therapy are administered concurrently. In some aspects, the anti-PD-1 binding protein or the anti-PD-L1 binding protein is administered after the chemoradiation therapy.

In some aspects of the combination for use, the combination extends progression free survival (PFS) in the patient. In some aspects of the combination for use, the combination increases the overall response rate (ORR) in the patient.

In an aspect, this disclosure provides a use of an anti-PD-1 binding protein or an anti-PD-L1 binding protein for the manufacture of a medicament for the treatment of cervical cancer in a patient. In some aspects, the medicament further comprises a chemoradiation therapy. In some aspects, the cervical cancer is locally advanced cervical cancer (LACC). In some aspects, the patient has a tumor area positivity (TAP) score of ≥ 20% for PD-L1 expression.

In some aspects of the use, the anti-PD-1 binding protein or the anti-PD-L1 binding protein is an anti-PD-1 antibody selected from pembrolizumab, nivolumab, and cemiplimab. In some aspects, the anti-PD-1 binding protein or the anti-PD-L1 binding protein is an anti-PD-L1 antibody selected from durvalumab, avelumab, atezolizumab, and sugemalimab. In some aspects, the anti-PD-1 binding protein or the anti-PD-L1 binding protein is a bispecific antibody, wherein the bispecific antibody is MEDI5752.

In some aspects of the use, the chemoradiation therapy comprises concurrent chemoradiation therapy. In some aspects, the chemoradiation therapy comprises external beam radiotherapy and brachytherapy. In some aspects, the chemoradiation therapy comprises a platinum-based chemotherapy. In some aspects, the platinum-based chemotherapy is cisplatin or carboplatin.

In some aspects of the use, treatment comprises administering 1500 mg of the anti-PD-L1 antibody to the patient intravenously every four weeks (Q4W). In some aspects, the treatment comprises administering 1500 mg of the anti-PD-L1 antibody to the patient intravenously every three weeks (Q3W). In some aspects, the anti-PD-L1 antibody is durvalumab.

In some aspects of the use, the treatment comprises administering 500 mg or 750 mg of a bispecific antibody to the patient intravenously every three weeks (Q3W). In some aspects, the bispecific antibody is MEDI5752.

In some aspects of the use, the anti-PD-1 binding protein or the anti-PD-L1 binding protein, and the chemoradiation therapy are administered concurrently. In some aspects, the anti-PD-1 binding protein or the anti-PD-L1 binding protein is administered after the chemoradiation therapy.

In some aspects of the use, the treatment extends progression free survival (PFS) in the patient. In some aspects of the use, the treatment increases the overall response rate (ORR) in the patient.

In an aspect, this disclosure provides a method of treating locally advanced cervical cancer (LACC) in a patient, wherein the method comprises administering 1500 mg of durvalumab to the patient intravenously every four weeks (Q4W), wherein the patient has a tumor area positivity (TAP) score of ≥ 20% for PD-L1 expression.

In an aspect, this disclosure provides a method of treating locally advanced cervical cancer (LACC) in a patient, wherein the method comprises administering 500 mg or 750 mg of MEDI5752 to the patient intravenously every three weeks (Q3W), wherein the patient has a tumor area positivity (TAP) score of ≥ 20% for PD-L1 expression.

In some aspects of the methods as disclosed herein, the method of either claim 61 or claim 62, wherein the method further comprises administration of a chemoradiation therapy. In some aspects, the chemoradiation therapy comprises concurrent chemoradiation therapy. In some aspects, the chemoradiation therapy comprises external beam radiotherapy and brachytherapy. In some aspects, the chemoradiation therapy comprises a platinum-based chemotherapy. In some aspects, the platinum-based chemotherapy is cisplatin or carboplatin.

In some aspects of the methods as disclosed herein, durvalumab or MEDI5752 and the chemoradiation therapy are administered concurrently. In some aspects, durvalumab or MEDI5752 is administered after the chemoradiation therapy.

In some aspects of the methods as disclosed herein, the method extends progression free survival (PFS) in the patient. In some aspects of the methods as disclosed herein, the method increases the overall response rate (ORR) in the patient.

In an aspect, this disclosure provides a combination of durvalumab and a chemoradiation therapy for the treatment of locally advanced cervical cancer (LACC) in a patient, wherein 1500 mg of durvalumab is administered to the patient intravenously every four weeks (Q4W), and wherein the patient has a tumor area positivity (TAP) score of ≥ 20% for PD-L1 expression.

In an aspect, this disclosure provides a combination of MEDI5752 and a chemoradiation therapy for the treatment of locally advanced cervical cancer (LACC) in a patient, wherein 500 mg or 750 mg of MEDI5752 is administered to the patient intravenously every three weeks (Q3W), and wherein the patient has a tumor area positivity (TAP) score of ≥ 20% for PD-L1 expression.

In some aspects of the combinations as disclosed herein, the chemoradiation therapy comprises concurrent chemoradiation therapy. In some aspects of the combinations as disclosed herein, the chemoradiation therapy comprises external beam radiotherapy and brachytherapy. In some aspects of the combinations as disclosed herein, the chemoradiation therapy comprises a platinum-based chemotherapy. In some aspects of the combinations as disclosed herein, the platinum-based chemotherapy is cisplatin or carboplatin.

In some aspects of the combinations as disclosed herein, durvalumab or MEDI5752 is administered after the chemoradiation therapy.

In some aspects of the combinations as disclosed herein, the combination extends progression free survival (PFS) in the patient. In some aspects of the combinations as disclosed herein, the combination increases the overall response rate (ORR) in the patient.

### BRIEF DESCRIPTION OF THE DRAWINGS

The accompanying drawings are included to provide a further understanding of the methods, combinations, and uses of the disclosure. The drawings illustrate one or more aspects of the disclosure, and together with the description serve to explain the principles and operation of the disclosure.
**FIG. 1** shows progression-free survival (PFS) of patients from the CALLA study. Progression-free survival, as per RECIST 1.1 as assessed by investigator or histopathologic confirmation of local tumor progression, Kaplan-Meier plot (full analysis set). CI=confidence interval; CRT=chemoradiotherapy; PFS=progression-free survival; RECIST=Response Evaluation Criteria in Solid Tumors.

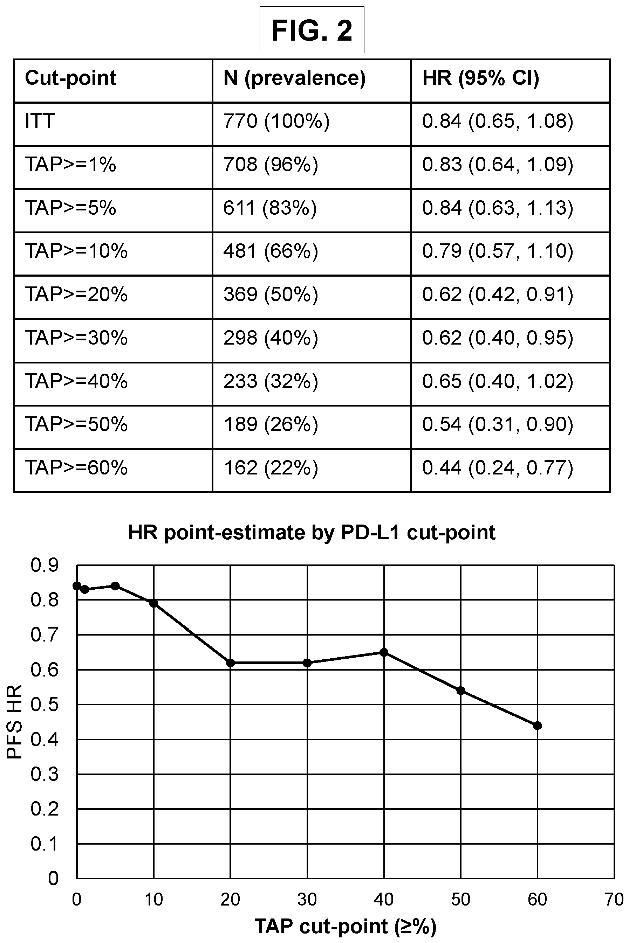

**FIG. 2** shows results from post-hoc analysis of the CALLA study. A PFS benefit was observed for patients with tumor PD-L1 TAP ≥ 20% who received durvalumab + CRT versus placebo + CRT with increasing PD-L1 cut-point.
**FIG. 3** shows results from post-hoc analysis of the CALLA study. A PFS benefit was observed for patients with tumor PD-L1 TAP ≥ 20% who received durvalumab + SoC CCRT (n=191) versus placebo + SoC CCRT (n=178) (hazard ratio, 0.62 [95% CI, 0.42-0.91]). CCRT = concurrent chemoradiation.
**FIG. 4** shows that a lower hazard ratio relative to the intent-to-treat population was observed for patients with lymph node-positive disease (Stage IB2-IVA) in the durvalumab arm (n=277) versus placebo (n=278) (0.77 [95% CI, 0.58-1.03]), whereas patients with lymph node-negative disease (Stage ≥III) had no PFS benefit with durvalumab (hazard ratio, 1.11 [95% CI, 0.65-1.91]).
**FIG. 5** shows that patients with tumor PD-L1 TAP ≥ 20%, lower hazard ratios were observed regardless of lymph node status (lymph node positive: durvalumab, n=138; placebo, n=141; hazard ratio, 0.66 [95% CI, 0.42-1.01]; lymph node negative: durvalumab, n=53; placebo, n=37; hazard ratio, 0.60 [95% CI, 0.26-1.41]).

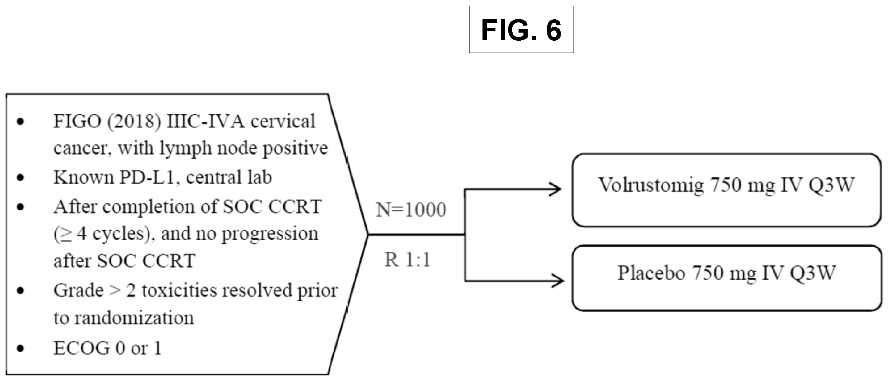

**FIG. 6** shows the study design for the treatment of cervical cancer with MEDI5752. Volrustomig/placebo until clinical or radiological progression, or up to 24 months. Thereafter, dependent on investigator's opinion if benefiting from treatment and do not meet any other discontinuation criteria. Abbreviations: CCRT=concurrent chemoradiation therapy; ECOG=Eastern Cooperative Oncology Group; FIGO=The International Federation of Gynecologists and Obstetricians; IV=intravenous; N=number of participants randomized; PD-L1=programmed death-ligand 1; R=randomization; SOC=standard of care; Q3W=every three weeks.

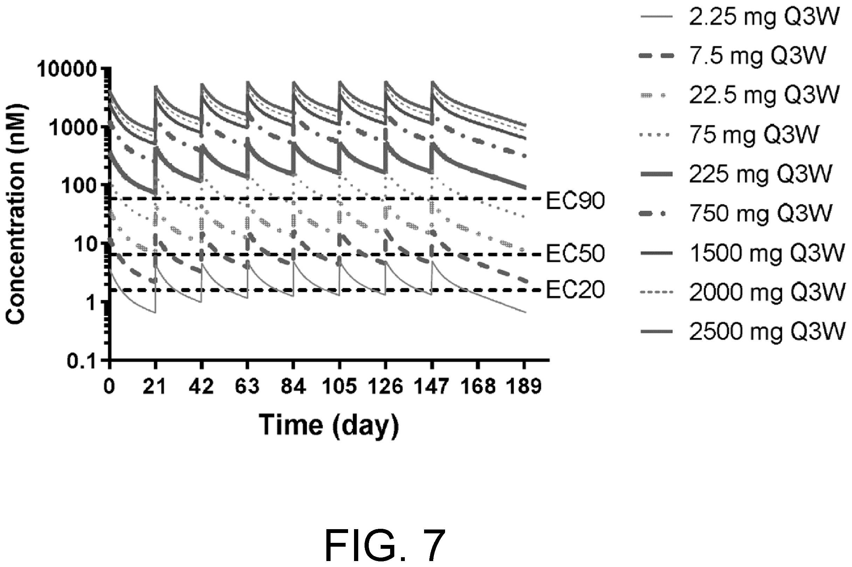

**FIG. 7** shows the predicted MEDI5752 concentration-time profiles following IV infusion of MEDI5752 Q3W with projected EC₂₀, EC₅₀, and EC₉₀ for PD-1.

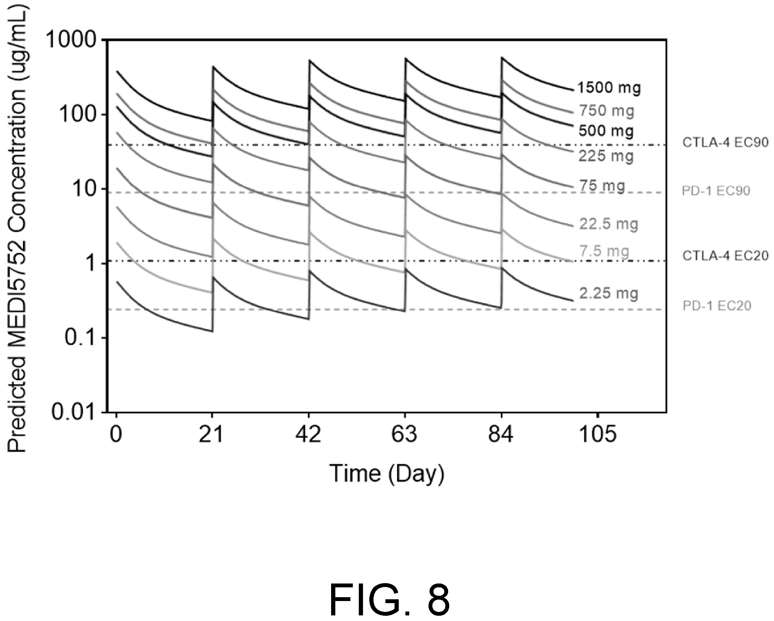

**FIG. 8** shows the predicted MEDI5752 concentration-time profiles following IV infusion of MEDI5752 Q3W with projected EC₂₀, and EC₉₀ for CTLA-4 and EC₂₀, and EC₉₀ PD-1.
**FIGs. 9A-9C** show peripheral blood T cell proliferation (CD4+ Ki67+) (FIG. 9A) and (FIG. 9C) and T cell activation (ICOS expression on CD4 T cells) (FIG. 9B) as measured by flow cytometry following MEDI5752 as monotherapy or in combination with chemotherapy.
**FIG. 10A-10C** show total of expanded T cell clones (FIG. 10A) and proportion of newly expanded T cell clones (FIGs. 10B and 10C) as measured by T cell receptor sequencing (TCRseq) following MEDI5752 as monotherapy or in combination with chemotherapy.
**FIG. 11** shows percent free PD1 on CD4 T cells (PD1 Receptor occupancy measured longitudinally by flow cytometry following MEDI5752 administration).
**FIG. 12** shows the median duration of response for MEDI5752 monotherapy at various doses.
**FIG. 13** shows the objective responses of MEDI5752 monotherapy in diverse IO-naïve tumors across a range of MEDI5752 doses.
**FIG. 14** shows mean MEDI5752 pharmacokinetic profiles following IV infusion of MEDI5752 Q3W.
**FIG. 15** shows Exposure (C_{trough}) v. CD4 T cell proliferation following MEDI5752 treatment.

Skilled artisans will appreciate that elements in the Figures are illustrated for simplicity and clarity and have not necessarily been drawn to scale. For example, the dimensions of some of the elements in the Figures can be exaggerated relative to other elements to help improve understanding of the aspects of the present disclosure.

### DETAILED DESCRIPTION

The disclosure relates to methods, combinations, and uses for treating patients who have cervical cancer, comprising administering to the patient an anti-PD-1 binding protein or an anti-PD-L1 binding protein, and in some aspects, a chemoradiation therapy. The disclosed methods, combinations, and uses for treatment can provide for substantial improvement in a patient's progression-free survival (PFS), overall response rate (ORR), and/or overall survival (OS).

Unless defined otherwise, all technical and scientific terms used herein have the meaning commonly understood by a person skilled in the art to which this disclosure belongs. The following references provide one of skill with a general definition of many of the terms used in this disclosure: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

In this disclosure, "comprises," "comprising," "containing" and "having" and the like can have the meaning ascribed to them in U.S. Patent law and can mean "includes," "including," and the like; "consisting essentially of" or "consists essentially" likewise have the meaning ascribed in U.S. Patent law and are open-ended, allowing for the presence of more than that which is recited so long as basic or novel characteristics of that which is recited are not changed by the presence of more than that which is recited, but excludes prior art aspects.

Unless specifically stated or obvious from context, the term "or," as used herein, is understood to be inclusive. Unless specifically stated or obvious from context, the terms "a," "an," and "the," as used herein, are understood to be singular or plural.

Unless specifically stated or obvious from context, the term "about," as used herein, is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Any compositions, combinations, methods, or uses provided herein can be combined with one or more of any of the other compositions, combinations, methods, and uses provided herein.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

Provided herein is a method, combination, or use for extending progression-free survival in a patient with cervical cancer, the method, combination, or use comprising treating the patient with an anti-PD-1 binding protein or an anti-PD-L1 binding protein (*e.g.,* an anti-PD-1 antibody, an anti-PD-L1 antibody, or an anti-PD-1/CTLA-4 bispecific antibody). In some aspects, the method, combination, or use further comprises a chemoradiation therapy. In some aspects, the cervical cancer is locally advanced cervical cancer (LACC).

Also provided herein is a method, combination, or use for increasing the overall response rate in a patient with cervical cancer, the method, combination, or use comprising treating the patient with an anti-PD-1 binding protein or an anti-PD-L1 binding protein (*e.g.,* an anti-PD-1 antibody, an anti-PD-L1 antibody, or an anti-PD-1/CTLA-4 bispecific antibody). In some aspects, the method, combination, or use further comprises a chemoradiation therapy. In some aspects, the cervical cancer is locally advanced cervical cancer (LACC).

Also provided herein is a method, combination, or use for treating a patient with cervical cancer, the method, combination, or use comprising treating the patient with an anti-PD-1 binding protein or an anti-PD-L1 binding protein (*e.g.,* an anti-PD-1 antibody, an anti-PD-L1 antibody, or an anti-PD-1/CTLA-4 bispecific antibody). In some aspects, the method further comprises a chemoradiation therapy. In some aspects, the cervical cancer is locally advanced cervical cancer (LACC).

In certain aspects of the methods, combinations, and uses disclosed herein, the patient has locally advanced cervical cancer with a tumor area positivity (TAP) score of greater than or equal to 20% of PD-L1 expression.

Programmed Death Ligand 1 ("PD-L1") is part of a complex system of receptors and ligands that are involved in controlling T-cell activation. Its normal function is to regulate the balance between T-cell activation and tolerance through interaction with its two receptors: programmed death 1 (also known as PD-1 or CD279) and CD80 (also known as B7-1 or B7.1). PD-L1 is also expressed by tumors and acts at multiple sites to help tumors evade detection and elimination by the host immune system. PD-L1 is expressed in a broad range of cancers with a high frequency.

Programmed Death-1 ("PD-1") is an approximately 31 kD type I membrane protein member of the extended CD28/CTLA-4 family of T cell regulators. PD-1 is expressed on activated T cells, B cells, and monocytes, where it serves as a receptor responsible for down-regulation of the immune system following activation by binding of PD-L1 or PD-L2. This process is exploited in many tumors via the over-expression of PD-L1, leading to a suppressed immune response.

The term "anti-PD-L1 binding protein" as used herein refers to polypeptides, binding proteins, antibodies, or antigen-binding fragments thereof that selectively bind a PD-L1 polypeptide. In some aspects, an anti-PD-L1 binding protein can be an anti-PD-L1 antibody. Exemplary anti-PD-L1 antibodies are described, for example, in U.S. Patent Nos. 8,779,108 and 9,493,565, which are incorporated herein by reference. In some aspects, the anti-PD-L1 antibody is durvalumab, avelumab, atezolizumab, or sugemalimab. In some aspects, the anti-PD-L1 antibody is durvalumab.

As used herein "durvalumab," refers to an antibody that selectively binds PD-L1 and blocks the binding of PD-L1 to PD-1 and CD80 receptors, as disclosed in U.S. Patent No. 9,493,565 (wherein durvalumab is referred to as "2.14H9OPT"), which is incorporated by reference herein in its entirety. The fragment crystallizable (Fc) domain of durvalumab contains a triple mutation in the constant domain of the IgG1 heavy chain that reduces binding to the complement component C1q and the Fcγ receptors responsible for mediating antibody-dependent cell-mediated cytotoxicity ("ADCC"). Durvalumab can relieve PD-L1-mediated suppression of human T-cell activation *in vitro* and inhibits tumor growth in a xenograft model via a T-cell dependent mechanism.

The term "anti-PD-1 binding protein" as used herein refers to polypeptides, binding proteins, antibodies, or antigen-binding fragments thereof that selectively bind a PD-1 polypeptide. In some aspects, an anti-PD-1 binding protein can be an anti-PD-1 antibody. In some aspects, the anti-PD-1 antibody is pembrolizumab, nivolumab, or cemiplimab.

In some aspects, an anti-PD-1 binding protein can refer to a bispecific antibody that selectively binds a PD-1 polypeptide as well as a second target polypeptide. In some aspects, an anti-PD-1 binding protein can refer to a bispecific antibody targeting both PD-1 and CTLA-4. In some aspects, the bispecific antibody targeting both PD-1 and CTLA-4 is MEDI5752.

MEDI5752 is a monovalent bispecific antibody targeting PD-1 and CTLA-4. In some aspects, the patient is administered one or more doses of MEDI5752, wherein the dose is a fixed dose of 500 mg or 750 mg. In some aspects, the patient is administered 500 mg or 750 mg of MEDI5752 bispecific antibody every three weeks. *See also* International Publication No. WO 2017/193032; U.S. Patent Application Publication No. US 2018/0022807; U.S. Patent No. 10,457,732; and U.S. Patent Application Publication Nos. US 2020/0172622 and US 2022/0251204, each of which is incorporated by reference herein in its entirety.

As used herein, the term "MEDI5752" refers to an anti-PD-1/CTLA-4 bispecific antibody that comprises the light chain of SEQ ID NO: 1 and the heavy chain of SEQ ID NO: 2 (PD-1) and the light chain of SEQ ID NO: 3 and the heavy chain of SEQ ID NO: 4 (CTLA-4). MEDI5752 is disclosed in U.S. Patent No. 10,457,732, which is herein incorporated by reference in its entirety (*see also* Tables 1-4). MEDI5752 is also referred to herein as "Volrustomig". Provided herein are methods, combinations, and uses for treating cancers in a subject (*e.g.*, a human subject) comprising administering to the subject antibodies (*e.g.*, bispecific antibodies, monoclonal antibodies, such as chimeric antibodies, humanized antibodies, or human antibodies) and antigen-binding fragments thereof which specifically bind to PD-1 and CTLA-4, (*e.g.*, human PD-1 and CTLA-4). In some aspects, PD-1 and CTLA-4, (*e.g.*, human PD-1 and CTLA-4) antibodies and antigen-binding fragments thereof that can be used in the methods, combinations, and uses provided herein include MEDI5752, a monovalent bispecific humanized immunoglobulin G1 (IgG1) monoclonal antibody (mAb) with an engineered fragment crystallizable (Fc) domain to reduce Fc effector function, that specifically binds PD-1 and CTLA-4.

MEDI5752 was constructed on the backbone of the DuetMab molecule. The DuetMab design is described in Mazor et al., MAbs. 7(2): 377-89 (2015), which is hereby incorporated by reference in its entirety. The "DuetMab," design includes knobs-into-holes (KIH) technology for heterodimerization of 2 distinct heavy chains and increases the efficiency of cognate heavy and light chain pairing by replacing the native disulfide bond in one of the CH1-CL interfaces with an engineered disulfide bond.

The Fc domain of MEDI5752 carries the triple mutations (TM) (L234F, L235E and P331S) designed to reduce Fc-mediated immune effector functions (Oganesyan et al, Acta Crystallogr. D. Biol. Crystallogr. 64(Pt 6): 700-04 (2008)). MEDI5752 includes anti-PD-1 and anti-CTLA-4 Fabs, engineered interchain disulfide in the anti-CTLA-4 CH1-CL interface and knob-into-hole IgG1-TM Fc. MEDI5752 includes a knob mutation in the heavy chain comprising a variable region that binds to CTLA-4 and the hole mutation in the heavy chain comprising a variable region that binds to PD-1.

In some aspects of the present disclosure, a bispecific antibody or antigen-binding fragment thereof for use in the methods, combinations, and uses described herein specifically binds to human PD-1 and human CTLA-4 and comprises the six CDRs of the MEDI5752 antibody listed as provided in Table 1.

**Table 1. VH CDR Amino Acid Sequences ¹**

| **Anti-body** | **VH CDR1 (SEQ ID NO:)** | **VH CDR2 (SEQ ID NO:)** | **VH CDR3 (SEQ ID NO:)** |
|---|---|---|---|
| MEDI5752 PD-1 | GFTFSDYGMH (SEQ ID NO: 8) | YISSGSYTIYSADSVKG (SEQ ID NO: 9) | RAPNSFYEYYFDY (SEQ ID NO: 10) |
| MEDI5752 CTLA-4 | GFTFSSYGMH (SEQ ID NO: 14) | VIWYDGSNKYYADSVKG (SEQ ID NO: 15) | DPRGATLYYYYYGMDV (SEQ ID NO: 16) |

| | | | |
|---|---|---|---|
| ¹The VH CDRs in Table 1 are determined according to Kabat. | | | |

**Table 2. VL CDR Amino Acid Sequences ²**

| **Anti-body** | **VL CDR1 (SEQ ID NO:)** | **VL CDR2 (SEQ ID NO:)** | **VL CDR3 (SEQ ID NO:)** |
|---|---|---|---|
| MEDI5752 PD-1 | SASSKHTNLYWSRHMYWY (SEQ ID NO: 5) | TSNRAT (SEQ ID NO: 6) | QQWSSNP (SEQ ID NO: 7) |
| MEDI5752 CTLA-4 | RASQSINSYLD (SEQ ID NO: 11) | AASSLQS (SEQ ID NO: 12) | QQYYSTP (SEQ ID NO: 13) |

| | | | |
|---|---|---|---|
| ²The VL CDRs in Table 2 are determined according to Kabat. | | | |

In some aspects of the present disclosure, a bispecific antibody or antigen-binding fragment thereof for use in the methods, combinations, and uses described herein specifically binds to human PD-1 and CTLA-4 and comprises the variable heavy chain (VH) and variable light chain (VL) of the MEDI5752 antibody.

In some aspects of the present disclosure, a bispecific antibody or antigen-binding fragment thereof for use in the methods, combinations, and uses described herein specifically binds to human PD-1 and CTLA-4 and comprises the Heavy Chain (HC) of the MEDI5752 antibody listed in Table 3.

**Table 3: Full-length heavy chain amino acid sequences**

| **Antibody** | **Amino Acid Sequence (SEQ ID NO)** |
|---|---|
| MEDI5752 PD-1 | EVQLVESGGGLVQPGGSLRLSCAASGFTFSDYGMHWVRQAPGKGLEWVAYISSGSY TIYSADSVKGRFTISRDNAKNSLYLQMNSLRAEDTAVYYCARRAPNSFYEYYFDYW GQGTTVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKRVEPKS CDKTHTCPPCPAPEFEGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAS IEKTISKAKGQPREPQVCTLPPSREEMTKNQVSLSCAVKGFYPSDIAVEWESNGQP ENNYKTTPPVLDSDGSFFLVSKLTVDKSRWQQGNVFSCSVMHEALHNHYTQKSLSL SPGK (SEQ ID NO: 2) |
| MEDI5752 CTLA-4 | QVQLVESGGGVVQPGRSLRLSCAASGFTFSSYGMHWVRQAPGKGLEWVAVIWYDGS NKYYADSVKGRFTISRDNSKNTLYLQMNSLRAEDTAVYYCARDPRGATLYYYYYGM DVWGQGTTVTVSSASTKGPSVCPLAPSSKSTSGGTAALGCLVKDYFPEPVTVSWNS GALTSGVHTFPAVLQSSGLYSLSSVVTVPSSSLGTQTYICNVNHKPSNTKVDKRVE PKSVDKTHTCPPCPAPEFEGGPSVFLFPPKPKDTLMISRTPEVTCVVVDVSHEDPE VKFNWYVDGVEVHNAKTKPREEQYNSTYRVVSVLTVLHQDWLNGKEYKCKVSNKAL PASIEKTISKAKGQPREPQVYTLPPCREEMTKNQVSLWCLVKGFYPSDIAVEWESN GQPENNYKTTPPVLDSDGSFFLYSKLTVDKSRWQQGNVFSCSVMHEALHNHYTQKS LSLSPGK (SEQ ID NO: 4) |

**Table 4: Full-length light chain amino acid sequences**

| **Antibody** | **Amino Acid Sequence (SEQ ID NO)** |
|---|---|
| MEDI5752 PD-1 | QIVLTQSPATLSLSPGERATLSCSASSKHTNLYWSRHMYWYQQKPGQAPRLLIYLT SNRATGIPARFSGSGSGTDFTLTISSLEPEDFAVYYCQQWSSNPFTFGQGTKLEIK RTVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC (SEQ ID NO: 1) |
| MEDI5752 CTLA-4 | DIQMTQSPSSLSASVGDRVTITCRASQSINSYLDWYQQKPGKAPKLLIYAASSLQS GVPSRFSGSGSGTDFTLTISSLQPEDFATYYCQQYYSTPFTFGPGTKVEIKGQPKA APSVTLFPPCSEELQANKATLVCLISDFYPGAVTVAWKADSSPVKAGVETTTPSKQ SNNKYAASSYLSLTPEQWKSHRSYSCQVTHEGSTVEKTVAPTEVS (SEQ ID NO: 3) |

As generally used herein, the terms "treat," "treating," "treatment," and the like, refer to reducing, ameliorating, or slowing the progression of a disorder or disease and/or symptoms associated with a disorder or disease. It will be appreciated that, although not precluded, treating a disorder, disease, or condition does not require that the disorder, disease, or condition or associated symptoms be completely eliminated. In some aspects relating to locally advanced cervical cancer, "treat," "treating," "treatment," can refer to achieving any one or combination of primary or secondary clinical endpoints.

In some aspects of the methods, combinations, and uses disclosed herein, the patient has locally advanced cervical cancer with a tumor area positivity (TAP) score of greater than or equal to 20% of PD-L1 expression. The PD-L1 expression can be measured by Tumor Area Positivity (TAP) score using the Ventana PD-L1 (SP263) Assay. As used herein, a "TAP score" is defined as a combination of tumor cell area with PD-L1 expression and immune cell area with PD-L1 expression. In some aspects, a "TAP20%" score can be PD-L1 staining of any intensity in tumor cell membranes and tumor-associated immune cells covering 20% or more of tumor area.

The anti-PD-1 binding protein or the anti-PD-L1 binding protein (*e.g.,* an anti-PD-L1 antibody, an anti-PD-1 antibody, or an anti-PD-1/CTLA-4 bispecific antibody) can be administered once every four weeks while providing benefit to the patient. In some aspects, the patient is administered additional follow-on doses. Follow-on doses can be administered at various time intervals depending on the patient's age, weight, clinical assessment, tumor burden, and/or other factors, including the judgment of the attending physician.

The anti-PD-1 binding protein or the anti-PD-L1 binding protein (*e.g.,* an anti-PD-L1 antibody, an anti-PD-1 antibody, or an anti-PD-1/CTLA-4 bispecific antibody) can be administered once every three weeks while providing benefit to the patient. In some aspects, the patient is administered additional follow-on doses. Follow-on doses can be administered at various time intervals depending on the patient's age, weight, clinical assessment, tumor burden, and/or other factors, including the judgment of the attending physician.

In some aspects, multiple doses of anti-PD-1 binding protein or the anti-PD-L1 binding protein (*e.g.,* an anti-PD-L1 antibody, an anti-PD-1 antibody, or an anti-PD-1/CTLA-4 bispecific antibody) are administered to the patient. In some aspects, at least three doses, at least four doses, at least five doses, at least six doses, at least seven doses, at least eight doses, at least nine doses, at least ten doses, at least fifteen doses, at least twenty-six doses, or more than at least twenty doses can be administered to the patient. In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody) is administered over a two-week period, over a four-week treatment period, over a six-week treatment period, over an eight-week treatment period, over a twelve-week treatment period, over a twenty-four-week treatment period, over a one-year treatment period, or more than over a one-year treatment period.

In some aspects, the interval between doses can be every four weeks (Q4W). In some aspects, the intervals between doses can be every two months.

In some aspects, the interval between doses can be every three weeks (Q3W). In some aspects, the intervals between doses can be every two months.

In some aspects, the patient is administered one or more doses of an anti-PD-L1 antibody, wherein the dose is a fixed dose of 1500 mg. In some aspects, the patient is administered 1500 mg of the anti-PD-L1 antibody every four weeks. In some aspects, the patient is administered one or more doses of the anti-PD-L1 antibody wherein the dose is about 20 mg/kg. In some aspects, the patient is administered 20 mg/kg of the anti-PD-L1 antibody every four weeks.

In some aspects, the patient is administered one or more doses of the anti-PD-1/CTLA-4 bispecific antibody, wherein the dose is a fixed dose of 500 mg or 750 mg. In some aspects, the patient is administered 500 mg or 750 mg of the anti-PD-1/CTLA-4 bispecific antibody every three weeks. In some aspects, the patient is administered MEDI5752 at 500 mg or 750 mg every three weeks (Q3W). In some aspects, the patient is administered one or more doses of the anti-PD-1/CTLA-4 bispecific antibody, wherein the dose is a fixed dose of 500 mg or 750 mg. In some aspects, the patient is administered 500 mg or 750 mg of the anti-PD-1/CTLA-4 bispecific antibody every three weeks. In some aspects, the patient is administered MEDI5752 at 500 mg or 500 mg every three weeks (Q3W).

In some aspects, administration of anti-PD-1 binding protein or the anti-PD-L1 binding protein (*e.g.,* an anti-PD-L1 antibody, an anti-PD-1 antibody, or an anti-PD-1/CTLA-4 bispecific antibody) according to the methods, combinations, and uses provided herein is through parenteral administration. For example, the anti-PD-1 binding protein or the anti-PD-L1 binding protein (*e.g.,* an anti-PD-L1 antibody, an anti-PD-1 antibody, or an anti-PD-1/CTLA-4 bispecific antibody) can be administered by intravenous infusion. In some aspects, the administration is by intravenous infusion.

The methods, combinations, and uses for treatment disclosed herein further comprise chemoradiation therapy (CRT) or chemoradiotherapy. In one embodiment, chemoradiation therapy comprises concurrent chemoradiation therapy (CCRT). Concurrent chemoradiation therapy occurs wherein both radiotherapy and chemotherapy are administered concurrently (for example by simultaneous (same day) administration) during a single phase (Concurrent Chemoradiation Therapy or CCRT). In some aspects, chemoradiation therapy comprises one or more platinum-based chemotherapeutic agents. In some aspects, the one or more platinum-based chemotherapeutic agents is carboplatin, cisplatin, oxaliplatin, or combinations thereof. In some aspects, chemoradiation therapy comprises cisplatin or carboplatin. In some aspects, the chemoradiation therapy comprises external beam radiotherapy and/or brachytherapy.

In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody) is administered concurrently with chemoradiation therapy. In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody), and chemoradiation therapy are administered within about three days of each other. In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody), and chemoradiation therapy are administered within about two days of each other. In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody), and chemoradiation therapy are administered within about one day of each other. In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody), and chemoradiation therapy are administered concurrently (for example by simultaneous (same day) administration). In some aspects, the anti-PD-1 binding protein or the anti-PD-L1 binding protein (*e.g.,* an anti-PD-L1 antibody, an anti-PD-1 antibody, or an anti-PD-1/CTLA-4 bispecific antibody) is administered on Cycle 1 Day 1 of the chemoradiation therapy.

In some aspects, the methods, combinations, and uses for treatment disclosed herein provide an increase in progression-free survival (PFS) relative to placebo. In some aspects, the methods, combinations, and uses provide an increase in objective response rate (ORR) relative to placebo. In some aspects, the methods, combinations, and uses provide an increase in overall survival (OS) versus placebo.

Overall Survival (OS) relates to the time period beginning on the date of treatment until death due to any cause. OS may refer to overall survival within a period of time such as, for example, 12 months, 18 months, 24 months, and the like. Such periods of time can be identified, for example, as "OS24" which refers to the number (%) of patients who are alive at 24 months after treatment onset per the Kaplan-Meier estimate of overall survival at 24 months.

Progression-Free Survival (PFS) relates to the time period beginning on the date of treatment until the date of objective disease progression (RECIST 1.1) or death (by any cause in the absence of progression). In some aspects, the methods, combinations, and uses of the disclosure provide for increase in PFS. In some aspects, the methods, combinations, and uses provide for PFS of at least 9 months to at least about 24 months (*e.g.*, at least 9, 10, 11, 12, 13, 14, 15, 16, 17, 18, 19, 20, 21, 22, 23, 24, or more than 24 months, and up to about 5 years).

Objective Response Rate (ORR) refers to the number (%) of patients with at least one visit response of Complete Response (CR) or partial response (PR) per RECIST 1.1.

Cervical tumors are staged using FIGO (2009) classification, which is the most widely used classification (Haie-Meder et al., "Cervical cancer: ESMO Clinical practice guidelines for diagnosis, treatment and follow-up," Ann. Oncol. 21(5): v37-40 (2010)) and is one of the most important prognostic factors (Marth et al., "ESMO Clinical practice guidelines for diagnosis, treatment and follow-up," Ann. Oncol. 28(suppl_4): iv72-83 (2017)). Cervical cancer is the only gynecological cancer that is clinically staged based on tumor size, vaginal or parametrial involvement, bladder/rectum extension, and distant metastases. It requires examination under anaesthesia, radiological imaging such as chest X-ray and IV pyelogram, or other diagnostic tools. CT can detect pathological lymph nodes, MRI can determine tumor size, degree of stromal penetrations, parametrial involvement, vaginal extension, and corpus extension with high accuracy.

It is to be understood that the particular aspects of the specification are described herein are not limited to specific aspects presented and can vary. It also will be understood that the terminology used herein is for the purpose of describing particular aspects only and, unless specifically defined herein, is not intended to be limiting. Moreover, particular aspects disclosed herein can be combined with other aspects disclosed herein, as would be recognized by a skilled person, without limitation.

### EXAMPLES

The Examples that follow are illustrative of specific aspects of the disclosure, and various uses thereof. They are set forth for explanatory purposes only and should not be construed as limiting the scope of the disclosure in any way.

### Example 1: Efficacy of Durvalumab in Combination with Chemoradiation Therapy in Patients with Locally Advanced Cervical Cancer (LACC)

Concurrent chemoradiation therapy (CCRT) has been the standard of care for locally advanced cervical cancer (LACC) for over 20 years. However, 30%-40% of treated patients experience recurrence or progression within 5 years. Immune checkpoint inhibition has improved outcomes for patients with metastatic/recurrent cervical cancer. The benefit of adding durvalumab, a programmed cell death ligand 1 antibody, with and following (chemoradiation therapy) CRT for LACC was assessed. In a phase 3, randomized trial ("CALLA study"; Clinical Trials No.: NCT03830866), patients with previously untreated LACC (FIGO 2009 Stage IB2-IIB node positive, Stage ≥III any node status), were randomized (1:1) at 105 hospital sites across 15 countries via an interactive web response system to receive double-blind durvalumab (1500 mg intravenously once every four weeks) or placebo with and following CRT (CRT compliance was monitored). The primary endpoint was progression-free survival (PFS) and was assessed by the investigator using RECIST v1.1, in the intention-to-treat (ITT) population.

| **Table 5 Objectives and Endpoints** | |
|---|---|
| **Primary objective:** | **Endpoint/variable:** |
| To assess the efficacy of durvalumab + SoC CCRT compared with placebo + SoC CCRT in terms of PFS | Endpoints based on the investigator assessment according to RECIST 1.1 or histopathologic confirmation of local tumor progression: |
| | • PFS: Time from date of randomization until tumor progression or death due to any cause |

| **Secondary objectives:** | **Endpoints/variables:** |
|---|---|
| To further assess the efficacy of durvalumab + SoC CCRT compared with placebo + SoC CCRT in terms of PFS (3 year), PFS in PD-L1 positive patients, overall survival (OS) (key), OS in PD-L1 positive patients, ORR, CR rate, and DoR in Patients with a CR | • OS: Time from date of randomization until the date of death by any cause |
| | Endpoints based on the investigator assessment according to RECIST 1.1 or pathologic assessment of local disease progression |
| | • PFS (3 year): Proportion of patients alive and progression-free at 3 years |
| | • PFS in PD-L1 positive patients: Time from date of randomization until tumor progression or death due to any cause in patients who are PD-L1 positive (>=1%) based on either tumor or immune cell staining |
| | • OS in PD-L1 positive patients: Time from date of randomization until the date of death by any cause in patients who are PD-L1 positive (>=1%) based on either tumor or immune cell staining |
| | • ORR: The percentage of evaluable patients with an Investigator-assessed visit response of CR or PR |
| | • CR rate: Disappearance of all target and non-target lesions DoR in Patients with a CR: Time from date of first detection of CR until the date of objective disease progression according to RECIST 1.1 |
| To assess the effect of durvalumab + SoC CCRT compared with placebo + SoC CCRT on the incidence of local progression, distant disease progression, and secondary malignancy as the first documented progression event | • Incidence of Local Progression, Distant Disease Progression, and Secondary Malignancy: Number and percentage of patients who develop local progression, distant disease recurrence, or secondary malignancy |
| To assess disease-related symptoms and health related quality of life (HRQoL) in patients with cervical cancer treated with durvalumab + SoC CCRT compared with placebo + SoC CCRT using the core quality of life questionnaire (EORTC QLQ-C30) and core quality of life questionnaire cervical cancer module (CX24) | • Change from baseline in EORTC QLQ-C30 and EORTC CX24 |
| To assess the PK of durvalumab when in combination with CCRT | Blood concentration of durvalumab |
| To investigate the immunogenicity of durvalumab in both arms in combination with CCRT | Presence of ADAs |

| **Safety objective:** | **Endpoint/variable:** |
|---|---|
| To assess the safety and tolerability profile of durvalumab + SoC CCRT compared to placebo + SoC CCRT | AEs, laboratory findings, vital signs, and physical examinations, |

| **Exploratory objective:** | **Endpoint/variable:** |
|---|---|
| To collect blood and tissue samples for defining biological response to durvalumab +SoC CCRT compared to placebo +SoC CCRT for candidate markers that may correlate with likelihood of clinical benefit. | • Analysis of blood/tissue samples to assess exploratory biomarkers, which may include, but is not limited to ctDNA, mRNA signatures, CD8 by IHC, and Tumor Mutational Burden (TMB) |
| To assess treatment-related symptoms using the patient-reported outcomes version of the Common Terminology Criteria for Adverse Events (PRO-CTCAE) | • Specific treatment-related PRO-CTCAE symptoms |
| To assess how a patient perceives her overall change in cancer symptoms since the start of study treatment using the PGIC and overall severity of cancer symptoms using PGIS. | • PGIC and PGIS |
| To explore the impact of treatment and disease state on health status assessed by EuroQol five-dimensional five-level questionnaire (EQ-5D-5L) to support health economic analysis and health technology assessment | • EQ- 5D- 5L |
| To describe and evaluate resource use associated with durvalumab treatment and underlying disease | • Health resource utilization measures including hospitalization, outpatient visits, or emergency department visits |

A total of 770 women (durvalumab, n=385; placebo, n=385; 44% Hispanic, 39% Asian) were randomised. Durvalumab did not significantly improve PFS over placebo (hazard ratio for disease progression or death, 0.84; 95% CI, 0.65-1.08; P=0.174); 12-month PFS was 76.0% (293/385) with durvalumab and 73.3% (282/385) with placebo. Adverse events of grade 3 or higher occurred in 51.7% (199/385) versus 51.0% (196/384) of patients who received durvalumab versus placebo.

### Study design

The randomized, double-blind CALLA study was carried out in 105 hospital sites in 15 countries. The CALLA trial protocol and all amendments were approved by a local institutional review board or independent ethics committee at each study site. Good Clinical Practice guidelines, as established by the International Conference on Harmonization, and ethical considerations detailed in the Declaration of Helsinki and Council for International Organizations of Medical Sciences International Ethical Guidelines were followed in the execution of the trial. The study protocol is described briefly below and is registered with the U.S. National Library of Medicine ClinicalTrials database as NCT03830866.

### Treatments and treatment duration:

- Durvalumab 1500 mg via intravenous (IV) infusion was administered Q4W, starting day patients begin treatment (C1D1), until completion of planned treatment or progression of disease (PD).
- Placebo: Sterile solution of 0.9% (weight/volume [w/v]) sodium chloride for injection via IV infusion Q4W, starting on C1D1, until completion of planned treatment or PD.
- Chemoradiotherapy: Cisplatin. 40 mg/m² IV Q1W × 5 weeks.

### Order of Administration

After randomization to receive durvalumab/placebo + SoC CCRT, patients received durvalumab/placebo via IV infusion over 1 hour (± 5min). Durvalumab/Placebo administration was followed by SoC chemotherapy via IV infusion with radiation therapy given on the same day. If there was not enough time to administer cisplatin it was administered within 1 calendar day of the durvalumab infusion. This allowed for flexibility in timely administration of the treatment regimen, as radiation therapy and durvalumab/placebo can be given on C1D1, and cisplatin given on C1D2 and similarly for subsequent cycles.

Cisplatin should only be given on a day that external-beam radiation is scheduled. Radiation therapy can occur before or after durvalumab and the platinum agent are administered and should be given on the same day if possible. Cisplatin should not be given on a day of brachytherapy treatment.

All patients planned to receive 5 doses of cisplatin regardless of the radiation fractionation schedule planned. An additional 6th dose of platinum chemotherapy can be administered per investigator discretion.

Treatment with SoC CCRT was concurrent with durvalumab/placebo (*i.e*., starting on Cycle 1 Day 1). On days when both durvalumab/placebo and SoC CCRT are administered, durvalumab/placebo was administered first, followed by SoC chemotherapy.

### Radiation Therapy

This protocol requires photon external beam radiation therapy (EBRT), with either whole pelvic radiation therapy or extended field radiation therapy. 3D conformal techniques, and intensity modulated radiation therapy (IMRT) techniques followed by low dose rate (LDR), pulsed dose rate (PDR), or high dose rate (HDR) brachytherapy are allowed. 2D radiation is allowed only with pre approval from the study radiation oncology PI and study sponsor. All radiation therapy must be completed ideally within 56 days of initiation (less than or equal to 59 days for compliance).

The external beam radiation is given via conformal fields (3D conformal vs. 4 field design: AP/PA, opposed lateral fields) or IMRT, inclusive of the parametrial boost. In the case of extended field radiation therapy (EFRT) encompassing the pelvic and para-aortic lymph nodes (PALN), a 3D conformal plan can use an extended pelvic LN field to cover the PALN field. If field size to cover the target measures larger than institution's linear accelerator largest field size for clinical use, a two-isocenter technique is recommended to use for planning. The prescription dose was 45 Gy in 25 fractions at 1.8 Gy/fraction, unless a simultaneous integrated boost (SIB) was used for gross nodal disease with an IMRT technique. For SIB cases, the gross nodal PTV boost received 50 Gy in 2 Gy/fraction, and then received a sequential boost dose of 2 Gy per fraction for a total dose of 54-58Gy depending on the contribution of the brachytherapy and location of the lymph node or parametrial boosts. For conventional 3D conformal EBRT, the prescription dose was 45 Gy in 25 fractions at 1.8 Gy/fraction with a required parametrial boost and (optional based on disease involvement) lymph node boost.

The EBRT should be given once daily, 5 fractions per week. For brachytherapy, the prescription doses were 27.5 - 30 Gy for HDR and 35 - 40 Gy for LDR or PDR, following institutional protocol with image guided brachytherapy, or point based brachytherapy. Image guided brachytherapy, with 3D image based acquisition and volumetric planning by CT or MRI was strongly encouraged.

### Patients

Eligible patients were females aged ≥18 years with untreated histologically confirmed cervical adenocarcinoma, squamous carcinoma, or adenosquamous carcinoma; FIGO 2009 Stage IB2-IIB lymph node positive or FIGO 2009 Stages IIIA-IVA (any lymph node status); and no evidence of metastatic disease. A World Health Organization/Eastern Cooperative Oncology Group performance status score of 0 or 1 and adequate organ and marrow function were required for enrollment. Patients were required to have at least one lesion at baseline that was measurable per Response Evaluation Criteria in Solid Tumors (RECIST), version 1·1.14 Surgical or imaging-based nodal staging was acceptable, with pathological lymph node size defined by a short-axis diameter of ≥10 mm on the axial plane.

Patients who had undergone a previous hysterectomy or would undergo a hysterectomy as part of their initial cervical cancer therapy were excluded. Prior exposure to immune-mediated therapy for any indication or concurrent use of chemotherapy, durvalumab, biologic, or hormonal treatment for cancer were not permitted. Written informed consent was obtained from patients or their legal representatives before participation.

### Randomization and masking

Patients were randomized 1:1 to receive durvalumab or placebo concurrent with and following SoC CRT. Randomization was by central assignment using an interactive web response system and was stratified according to disease stage (FIGO Stage IB2-IIB and node positive, Stage ≥III and node negative, or Stage ≥III and node positive) and region of the world (United States, Canada, European Union, South Korea, Japan, or rest of world). Once a patient was confirmed as eligible for the study, the investigator or delegate obtained a unique randomization number for the patient and treatment assignment via the interactive web response system. Randomization codes were assigned sequentially within each stratum as patients became eligible for randomization. Blinded and unblinded access was controlled via the interactive web response system. Investigators remained blinded to each patient's assigned treatment throughout the study.

### Procedures

Patients received intravenous durvalumab 1500 mg or placebo once every 4 weeks (Q4W), concurrent with and following SoC CRT, for a total of 24 cycles. Chemoradiotherapy consisted of 45Gy external beam RT given at five fractions per week concurrent with intravenous cisplatin 40 mg/m² or carboplatin (area under the concentration-time curve) once weekly for five weeks (sixth dose optional), followed by image-guided intracavitary brachytherapy (high-dose rate, 27·5-30 Gy or low-dose/pulse-dose rate, 35-40 Gy). To ensure high-quality RT care was delivered, a global RT subcommittee reviewed and evaluated care for each patient, and a feasibility questionnaire was used to confirm compliance for each site. Treatment was continued until the maximum number of cycles was reached; histopathological, clinical, or radiological progression; unacceptable toxicity; withdrawal of consent; or noncompliance with the study protocol warranting withdrawal. Durvalumab dose delays, but not reductions, were permitted to manage toxicities. An independent data monitoring committee assessed ongoing safety analyses using unblinded safety data, and made recommendations to the sponsor regarding continuing, stopping, or modifying the study.

Disease progression was assessed by the investigator according to RECIST v1.1 or histopathologic confirmation of local tumor progression. Baseline tumor assessments were taken ≤28 days before randomization. Tumor radiological imaging, pelvic examination, and (if needed) biopsy for histopathological determination of tumor progression were performed at week 20 and every 12 weeks thereafter for 164 weeks after randomization, then every 24 weeks until progression or closure of the study. Images of the chest, abdomen, and pelvis were obtained by computed tomography (preferred) or magnetic resonance imaging, each with or without intravenous contrast.

For assessment of survival, patients were contacted every 3 months following treatment discontinuation. Information regarding the first and subsequent cancer therapies used after discontinuation of treatment were collected.

Safety and tolerability were assessed via adverse events, laboratory findings, vital signs, and physical examinations. Adverse events were graded according to the National Cancer Institute Common Terminology Criteria for Adverse Events (version 5). An immune-mediated adverse event was defined as an adverse event of special interest (AESI) that was associated with durvalumab exposure and was consistent with an immune-mediated mechanism of action without a clear alternate etiology. The European Organisation for Research and Treatment of Cancer (EORTC) Quality-of-Life Questionnaire Core 30 (QLQ-C30) and Cervical Cancer module (QLQ-CX24) were used to assess patient-reported quality of life.

Fresh or archival tumor biopsies (≤3 months old) were obtained at screening to measure pretreatment PD-L1 expression. PD-L1 expression was assessed at a central laboratory using the VENTANA PD-L1 (SP263) Assay (Ventana Medical Systems, Tucson, AZ, USA). PD-L1 expression was assessed according to the tumor area positivity (TAP) score, defined as the percentage of tumor area (tumor and desmoplasmic stroma) comprising tumor cells with membranous PD-L1 staining at any intensity and tumor-associated immune cells with any pattern of PD-L1 staining at any intensity.

### Definition of secondary endpoints

Overall response rate (ORR) was defined as the percentage of patients with at least one investigator-assessed overall visit response of complete response (CR) or partial response (PR). CR rate was defined as the percentage of patients with an overall visit response of CR. Confirmed ORR was defined as the percentage of patients with at least one confirmed visit response of CR or PR. Duration of response was defined as the time from the date of first documented response (CR or PR) until death in the absence of progression. For patients who did not progress, their duration of response was their progression-free survival censoring time. Health-related quality of life was assessed by change from baseline in European Organization for Research and Treatment of Cancer (EORTC) Quality-of-Life Questionnaire Core 30 (QLQ-C30) and EORTC Cervical Cancer 24(CX24)-item module.

### Summary of patient-reported quality of life study assessments

The EORTC QLQ-C30 and QLQ-CX24 were used to assess patient-reported quality of life. QLQ-C30 items are grouped into five functional and three symptom scales, five individual symptom items, and a two-item global measure of health. The QLQ-CX24 is a 24-item complementary module to the QLQ-C30, designed specifically for use in cervical cancer. Items are grouped into three-item multi-item scales and six single-item scales. The questionnaires were given at baseline and 2, 4, 8, 12, 16, and 20 weeks from the first treatment cycle, then every 12 weeks through week 164, every 24 weeks during weeks 165-260, and every 52 weeks thereafter. Results from the QLQ-C30 and QLQ-CX24 were evaluated using a mixed model for repeated measures analysis of change from baseline score for all post-baseline visits through the last scheduled visit for which at least 20 patients in each arm had a score. Treatment, visit, treatment-by-visit interaction, disease stage status, and region were included as fixed categorical effects. The baseline Global Health Status/Quality of Life (GHS/QoL) score and baseline GHS/QoL score by visit interaction were included as covariates.

### Outcomes

Progression-free survival (PFS) was the primary endpoint and defined as the time from date of randomization until tumor progression or death due to any cause. Overall survival was the key secondary endpoint and defined as the time from the date of randomization until death due to any cause regardless of whether the patient withdrew from randomized therapy or received another anti-cancer therapy. Other secondary endpoints included PFS in prespecified patient subgroups (*e.g.*, FIGO stage, chemotherapy received, PD-L1 TAP ≥1% and ≥5%, lymph node status), objective response rate (ORR), duration of response, and health-related quality of life. An exploratory post hoc analysis was also done to assess PFS in patients with tumor PD-L1 TAP ≥20%.

Additional secondary endpoints including PFS at 3 years, overall survival in patients with PD-L1-high expressing tumors, complete response rate, secondary malignancies, blood concentration of durvalumab, and presence of anti-durvalumab antibodies will be reported at a later date.

### Statistical analysis

Efficacy endpoints were analyzed in the intent-to-treat population (all randomized patients). The safety population (all patients who received at least one dose of durvalumab or placebo) was used for the safety analysis. The primary endpoint of progression-free survival (PFS), the key secondary endpoint of overall survival, and duration of response in patients with a complete response were estimated per the Kaplan-Meier method. The effect of durvalumab versus placebo was determined by a stratified log-rank test adjusted for disease stage and region of the world, with the magnitude of effect summarized with hazard ratio and 95% confidence interval (CI) using a stratified Cox proportional hazards model and Efron's method of tie handling. Between-arm differences in ORR were summarized by odds ratio using logistic regression models and associated 95% CI and P value. Change from baseline in the QLQ-C30 and QLQ-CX24 scales at each visit were analyzed using a mixed model for repeated measures. Adverse events observed up to 90 days following the last dose of study treatment were collected and summarized.

### Results

One thousand forty (1040) patients were enrolled from 105 sites in 15 countries. A total of 770 patients were randomly assigned to durvalumab (n=385) or placebo (n=385). Baseline patient demographics and disease characteristics were generally balanced across arms. The median patient age was 49 years (interquartile range [IQR], 41-57). Among the patient population, 339/770 (44.0%) identified as Hispanic/Latino and 300/770 (39.0%) were Asian. CALLA was mostly Stage III (461/770 [59.9%]) and a majority of patients had squamous histology (642/770 [83.4%]). At baseline, 91.9% of patients had a PD-L1 TAP score ≥1%, 79.4% had TAP >5%, and a majority had lymph node involvement (pelvic, 62.7%; para-aortic, 7.0%; both, 4.0%).

Median duration of follow-up from randomization to the data cut-off was 18.5 months (IQR, 13.2-21.5) for durvalumab and 18.4 months (13.2-23.7) for placebo. The median duration of treatment exposure was 15.8 months (IQR, 9.2-21.4) for durvalumab and 15.7 months (8.3-21.6) for placebo.

A total of 385/385 (100%) and 383/385 (99.5%) patients received CRT in the durvalumab and placebo groups, respectively (Table 6). Most patients received five or six cycles of cisplatin/carboplatin (335/385 [87.0%] for durvalumab; 346/384 [90.1%] for placebo). External beam radiation therapy (EBRT) was completed per protocol for 371/385 (96.4%) patients treated with durvalumab and 379/385 (98.4%) patients treated with placebo. Brachytherapy was completed per protocol for 363/385 (94.3%) and 360/385 (93.5%) patients. Median EBRT dose was 5400 (IQR, 5400-5800) cGy for the ITT population. Patients treated with durvalumab received a median of 4 (4-4) fractions of brachytherapy, while patients treated with durvalumab received 4 (4-5) fractions. Both treatment groups were treated with 700 (600-700) cGy per fraction of brachytherapy. Equivalent EQD2 dose was 8387 (8094-8649) cGy for patients treated with durvalumab and 8387 (8019-8714) cGy for patients treated with placebo. In the intent-to-treat population, of those variations to CRT delivery adjudicated by the CALLA RT Steering Committee, 42/215 (19.5%) and 57/230 (24.8%) events were considered potentially clinically relevant for the durvalumab arm and the placebo arm, respectively.

**Table 6. CRT treatment characteristics***

| | **Durvalumab (n=385)** | **Placebo (n=385)** | |
|---|---|---|---|
| Patients who received CRT | 385 (100) | 383 (99.5) | |
| Number of cycles of cisplatin/carboplatin | | | |
| | 5 | 199 (51.7) | 220 (57.1) |
| | 6 | 136 (35.3) | 126 (32.7) |
| EBRT delivered | | 385 (100) | 383 (99.5) |
| Brachytherapy delivered | | 366 (95.1) | 367 (95.3) |
| EBRT completed per protocol | | 371 (96.4) | 379 (98.4) |
| Brachytherapy completed per protocol | | 363 (94.3) | 360 (93.5) |
| Radiotherapy delivered in ≤59 days | | 278 (72.2) | 279 (72.5) |
| Radiotherapy delivered in >60 to ≤ 70 days | | 52 (13.5) | 51 (13.2) |
| Unless otherwise specified, data represent n (%) of patients. | | | |
| *Includes all patients who underwent randomisation (intention-to-treat population). | | | |
| **Japan with midline block reported. | | | |
| CRT=chemoradiotherapy; EBRT=external beam radiation therapy; IQR=interquartile range | | | |

**Table 7. Radiotherapy treatment characteristics at all study sites in Japan**

| | | **Durvalumab + CRT (n=28)** | **Placebo + CRT (n=24)** |
|---|---|---|---|
| Median (IQR) total EBRT dose | | | |
| | With midline block | 5400 cGy (5400-5400) (n=26) | 5400 cGy (5400-5520 (n=24) |
| | Without midline block | 5960 cGy (5440-6480) (n=2) | N/A (n=0) |

| | | **Durvalumab** + **CRT (n=27)** | **Placebo** + **CRT (n=24)** |
|---|---|---|---|
| Duration of EBRT + brachytherapy, median (IQR), days | | 50.0 (48.0-52.0) | 50.0 (47.0-51.5) |
| Brachytherapy no. of fractions, median (IQR) | | 4.0 (3.0-4.0) | 4.0 (3.0-4.0) |
| Brachytherapy dose per fraction, median (IQR), cGy | | 600 cGy (600-600) | 600 cGy (600-600) |
| Equivalent EQD2 dose, median (IQR), cGy | | 7027 (6540-7393) | 6926·5 (6555·5 -7273·5) |
| Patients with lymph node boost, n (%) | | 23 (85) | 17 (71) |
| Patients with parametrial boost, n (%) | | 2(7) | 2 (8) |
| Patients with para-aortic boost, n (%) | | 0 | 0 |
| BT=brachytherapy; EBRT=external beam radiation therapy; EQD2=equi-effective dose in 2 Gy per fraction; IQR=interquartile range; N/A=not applicable | | | |

Following the site qualification process for delivery of radiotherapy, nearly all (123/133 [92.5%]) study sites were approved to deliver RT. Patients received a median 56.0 (IQR, 51.0-60.0) days of RT (EBRT plus brachytherapy) in the durvalumab arm and 55.0 (51.0-60.0) days in the placebo arm (Table 8). Most patients in the global data set received intensity-modulated RT (627/717 [87.4%]) or three-dimensional conformal RT (88/717 [12.3%]), with one patient in the placebo arm receiving conventional two-dimensional RT (Table 9).

**Table 8. Radiotherapy delivery***

| | **Durvalumab + CRT n=357** | **Placebo + CRT n=360** |
|---|---|---|
| Duration of EBRT + brachytherapy, median (IQR), days | 56.0 (51.0-60.0) | 55.0 (51.0-60.0) |
| EBRT total dose, median (IQR), cGy† | 5400 (5400-5800) | 5400 (5400-5800) |
| Brachytherapy no. of fractions, median (IQR) | 4 (4-4) | 4 (4-5) |
| Brachytherapy dose per fraction, median (IQR), cGy‡ | 700 (600-700) | 700 (600-700) |
| Equivalent EQD2 dose, median (IQR), cGy§ | 8387 (8094-8649) | 8387 (8019-8714) |
| Subjects with lymph node boost, n (%) | 257 (72·0) | 259 (71·9) |
| Subjects with parametrial boost, n (%) | 307 (86·0) | 298 (82·8) |
| Subjects with para-aortic boost, n (%) | 123 (34·5) | 112 (31·1) |
| *Global data set, excluding Japan: from the safety analysis set, which includes one site in Japan that followed the Global Radiotherapy Protocol. | | |
| †In Japan, median total EBRT dose: with midline block (n=50/52), 5400 cGy; without midline block (n=2/52), 5960 cGy. | | |
| ‡In Japan, median brachytherapy dose per fraction: 600 cGy (n=51/52); median no. of fractions: 4.0 (n=51/52). | | |
| ^{§}In Japan, median equivalent EQD2 dose: 7027 cGy (durvalumab + CRT); 6926·5 cGy (placebo + CRT). | | |
| CRT=chemoradiotherapy; EBRT=external beam radiation therapy; EQD2=equi-effective dose in 2 Gy per fraction; IQR=interquartile range | | |

**Table 9. Radiotherapy key parameters**

| | | **Rest of world*** | | **Japan†** | |
|---|---|---|---|---|---|
| | | **Durvalumab + CRT n=357** | **Placebo + CRT n=360** | **Durvalumab + CRT n=28** | **Placebo** + **CRT n=24** |
| Radiotherapy used for EBRT | | | | | |
| | 3D conformal RT | 47 (13·2) | 41 (11·4) | 0 | 0 |
| | 3D conformal with contours | 0 | 0 | 28 (100) | 24 (100) |
| | Intensity-modulated radiation therapy | 310 (86·8) | 317 (88·1) | 0 | 0 |
| | Conventional 2D (approval of study PI and sponsor needed) | 0 | 1 (0·3) | 0 | 0 |

| Extended-field radiation therapy | | | | | |
|---|---|---|---|---|---|
| | Yes | 134 (37·5) | 112 (31·1) | 7(25·0) | 5 (20·8) |
| | No | 223 (62·5) | 247 (68·6) | 21 (75·0) | 19 (79·2) |
| Image-guided radiation therapy given daily per protocol | | | | | |
| | Yes | 284 (79·6) | 296 (82·2) | 23 (82·1) | 18 (75·0) |
| | No | 17 (4·8) | 12 (3·3) | 5 (17·9) | 6 (25·0) |
| Brachytherapy technique used | | | | | |
| | Standard (point-directed with 2D planar imaging) | 96 (26·9) | 84 (23·3) | 1 (3·6) | 3 (12·5) |
| | Standard (point-directed using volumetric brachytherapy imaging or planning for organs at risk) | 30 (8·4) | 31 (8·6) | 6 (21·4) | 5 (20·8) |
| | Volume-directed brachytherapy | 213 (59·7) | 228 (63·3) | 20 (71·4) | 16 (66·7) |
| Brachytherapy type (delivered) | | | | | |
| | High dose rate | 312 (87·4) | 317 (88·1) | 27 (96·4) | 24 (100) |
| | Low dose rate | 27 (7·6) | 26 (7·2) | 0 | 0 |
| | Pulsed dose rate | 0 (0) | 0 (0) | 0 | 0 |
| Data | represent n (%) patients. | | | | |
| *Global data set, excluding Japan: from the safety analysis set, which includes one site in Japan that followed the Global Radiotherapy Protocol. | | | | | |
| †One Japan site followed the Global Radiotherapy Protocol and is also included among the Japan sites. | | | | | |
| CRT=chemoradiotherapy; EBRT=external beam radiation therapy; PI=principal investigator; RT=radiation therapy | | | | | |

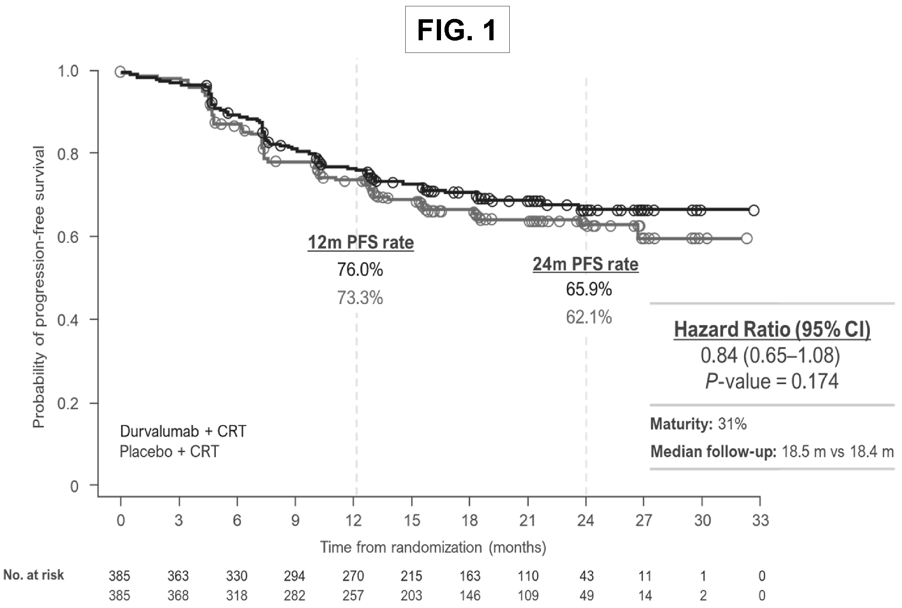

In the intent-to-treat population, durvalumab did not significantly improve PFS over placebo (hazard ratio for disease progression or death, 0.84; 95% CI, 0.65-1.08; P=0.174) (Fig. 1). The 12- and 24-month PFS for durvalumab versus placebo were 76.0% (293/385) versus 73.3% (282/385) and 65.9% (254/385) versus 62.1% (239/385), respectively. The upper limit of the 95% CI for the hazard ratio for disease progression or death was greater than 1 for all protocol-specified subgroups analyzed, including PD-L1 TAP ≥1% and ≥5% and pelvic and/or para-aortic lymph node involvement. Analyses of PFS by RT subgroup revealed no statistically significant differences between treatment arms. Formal significance testing was not performed for overall survival per the pre-specified multiple testing procedure. The hazard ratio for overall survival with durvalumab versus placebo was 0.78 (95% CI, 0.55-1.10; nominal P=0.156). The confirmed and unconfirmed ORR was 82.6% (318/385) in the durvalumab arm and 80.5% (310/385) in the placebo arm (odds ratio, 1.15 [95% CI, 0.80-1.66]). The percentage of patients with a confirmed complete response was 42.9% (165/385) in the durvalumab arm and 40.3% (155/385) in the placebo arm. The median duration of response was not reached for either treatment arm. Local disease recurrence was similar between the two groups, while a numerically higher number of patients exhibited distant disease progression events with placebo compared to durvalumab (Table 9).

A total of 371 patients in the durvalumab arm and 364 patients in the placebo arm had a tumor sample evaluable for PD-L1 expression. Similar proportions of patients in each arm had tumor PD-L1 TAP ≥1% at baseline (durvalumab, 92.5% [356/385]; placebo, 91.4% [352/385]). In patients with PD-L1 TAP ≥1%, PFS was similar in the durvalumab arm compared with the placebo arm (hazard ratio, 0.84; 95% CI, 0.64-1.10; P=0.203).

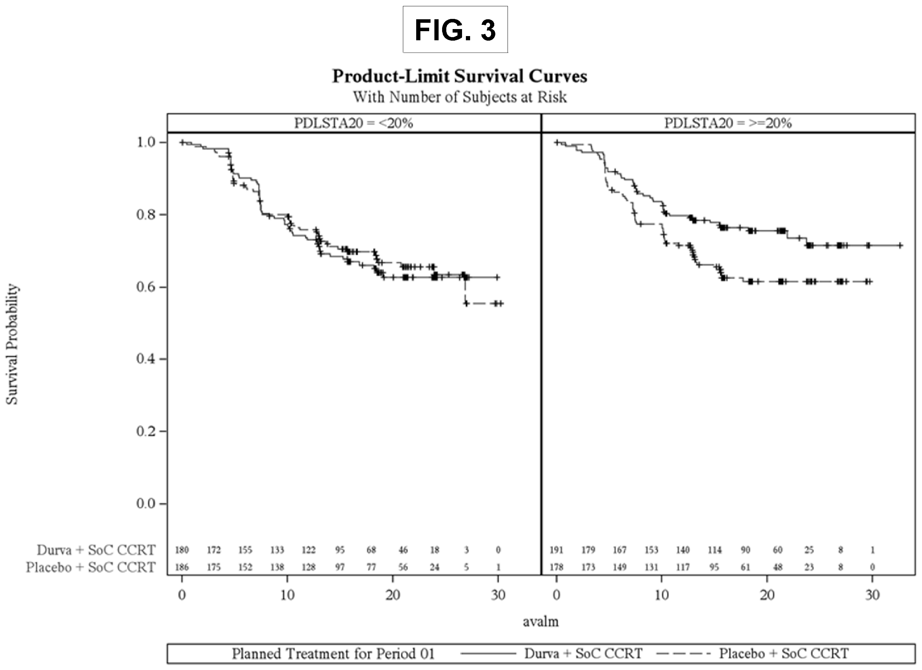

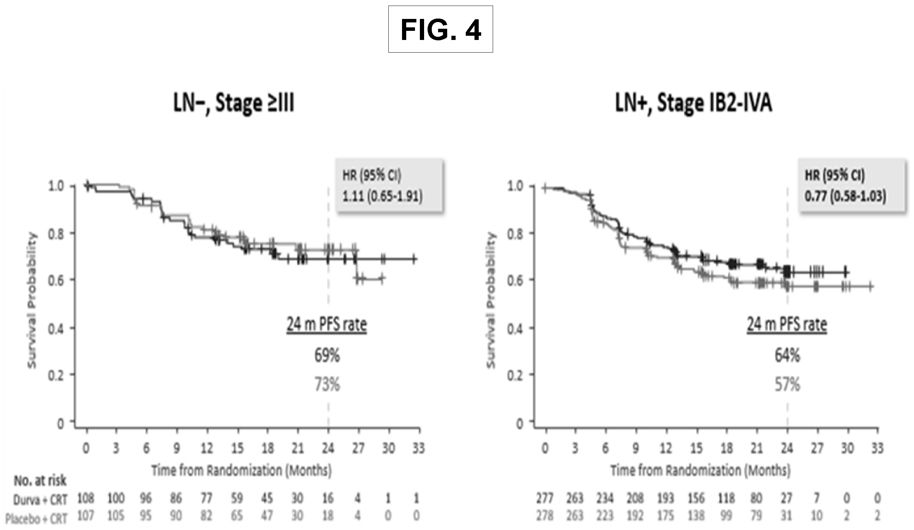

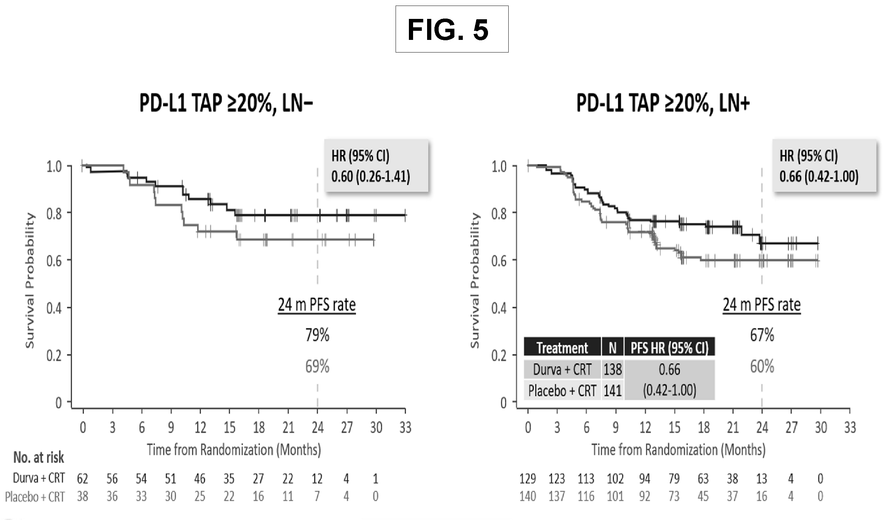

Unexpected and surprising results were obtained in an exploratory post hoc analysis. A PFS benefit was observed for patients with tumor PD-L1 TAP ≥20% who received durvalumab (n=191) versus placebo (n=178) (hazard ratio, 0.62 [95% CI, 0.42-0.91]) (Fig. 3). In contrast, patients with PD-L1 TAP <20% did not benefit from adding durvalumab to CRT (hazard ratio, 1.06 [95% CI, 0.74-1.53]). A lower hazard ratio relative to the intent-to-treat population was also observed for patients with lymph node-positive disease (Stage IB2-IVA) in the durvalumab arm (n=277) versus placebo (n=278) (0.77 [95% CI, 0.58-1.03]), whereas patients with lymph node-negative disease (Stage ≥III) had no PFS benefit with durvalumab (hazard ratio, 1.11 [95% CI, 0.65-1.91]) (Fig. 4). However, among patients with tumor PD-L1 TAP ≥20%, lower hazard ratios were observed regardless of lymph node status (lymph node positive: durvalumab, n=138; placebo, n=141; hazard ratio, 0.66 [95% CI, 0.42-1.01]; lymph node negative: durvalumab, n=53; placebo, n=37; hazard ratio, 0.60 [95% CI, 0.26-1.41]) (Fig. 5).

Compliance rates at baseline were 92.1% (708/769) for the EORTC QLQ-C30 questionnaire and 91.4% (703/769) for the QLQ-CX24 questionnaire in both treatment arms combined; rates declined over time but were 66.7% (166/249) and 66.3 % (165/249) in both arms combined at Cycle 24 Day 1, respectively. For the QLQ-C30 Global Health Status/Quality of Life scale, where a positive score indicates improvement, the adjusted mean change from baseline over all visits for the durvalumab and placebo arms was 2.1 (95% CI, 0.5-3.6) and 2.8 (95% CI, 1.2-4.3), respectively, and the estimated mean difference between durvalumab and placebo was -0.7 points (95% CI, -2.8 to 1.3; P=0.487). For the QLQ-CX24 symptom experience, where a negative score indicates improvement, the adjusted mean change from baseline over all visits for both the durvalumab and placebo arms was -7.6 (durvalumab 95% CI, -8.6 to -6.7; placebo 95% CI, -8.5 to -6.6; P=0.930). No significant differences were observed in symptom experience, role functioning, physical functioning, and quality of life between the durvalumab and placebo arms.

Adverse events of any cause occurred in 379/385 (98.4%) patients in the durvalumab arm and 377/384 (98.2%) in the placebo arm. Grade 3 or 4 adverse events occurred for 199/385 (51.7%) patients who received durvalumab and 196/384 (51.0%) who received placebo. The most common Grade 3 or 4 adverse events in both treatment arms were anemia, decreased white blood cell count, neutropenia, decreased neutrophil count, and lymphopenia. Immune-mediated adverse events occurred in 147/385 (38.2%) patients who received durvalumab and 91/384 (23.7%) patients who received placebo. AESIs for durvalumab occurred in 237/385 (61.6%) patients in the durvalumab arm and 221/384 (57.6%) patients in the placebo arm. The most common AESIs were diarrhea, hypothyroid events, hyperthyroid events, and rash in the durvalumab arm and diarrhea, rash, hypothyroid events, and colitis in the placebo arm.

The most common treatment-related adverse events were nausea, anemia, and diarrhea, which occurred in 193/385 (50.1%), 159/385 (41.3%), and 147/385 (38.2%) patients receiving durvalumab and 184/384 (47.9%), 154/384 (40.1%), and 161/384 (41.9%) receiving placebo, respectively. Treatment-related adverse events that led to discontinuation of study treatment occurred in 48/385 (12.5%) patients receiving durvalumab and 37/384 (9.6%) receiving placebo. Six patients died due to treatment-related adverse events of sepsis, urinary tract infection, blood loss anemia, endocrine disorder, and pulmonary embolism (durvalumab arm; n=1 each) and pneumonia (placebo arm; n=1). Sepsis and endocrine disorder were considered to be possibly caused by durvalumab and the remaining events were attributed to CRT.

RT-related toxicities occurring within 1 year of the last date of RT were experienced by 291/385 (75.6%; Grade ≥3, 116/385 [30.2%]) patients in the durvalumab arm and 287/384 (74.7%; Grade ≥3, 106/384 [27.6%]) in the placebo arm. The most common all-grade RT-related toxicities were diarrhea, anemia, and nausea, which were reported for 124/385 (32.2%), 106/385 (27.5%), and 71/385 (18.4%) patients, respectively, in the durvalumab arm and 135/384 (35.2%), 108/384 (28.1%), and 78/384 (20.3%) patients in the placebo arm. RT-related toxicities occurring more than 1 year after the last date of RT were experienced by 37/385 (9.6%; Grade ≥3, 7/385 [1.9%]) patients in the durvalumab arm and 36/384 (9.4%; Grade ≥3, 4/384 [1.0%]) in the placebo arm. In the durvalumab arm, the most common all-grade RT-related toxicities occurring more than 1 year after RT were rectal hemorrhage and gastroenteritis radiation, which were each reported for 5/385 (1.3%) patients. In the placebo arm, the most common RT-related toxicities in this time period were radiation proctitis (6/384 [1.6%]) and cystitis radiation (4/384 [1.0%]).

In the CALLA study, durvalumab combined with and following CRT did not significantly improve PFS compared with CRT alone for patients with high-risk LACC. However, durvalumab surprisingly confers a PFS benefit in patients with PD-L1 TAP ≥20% expression regardless of lymph node status based on post hoc analyses. Safety was similar between treatment arms, and no new or unexpected toxicities occurred. The CALLA study demonstrated the possibility of delivering high-quality, global RT, and RT delivery was not influenced by the addition of durvalumab. The CALLA study illustrates the importance of strong multidisciplinary collaboration and a quality control strategy to achieve optimal CRT delivery.

**Table 10. Baseline demographic and disease characteristics of the patients**

| | | **Durvalumab (n=385)** | **Placebo (n=385)** |
|---|---|---|---|
| Age (years) | | | |
| | Median | 50.0 | 48.0 |
| | IQR | 41.0-57.0 | 40.0-57.0 |

| Race or ethnic group, n (%) | | | |
|---|---|---|---|
| | Asian | 152 (39.5) | 148 (38.4) |
| | White | 130 (33.8) | 125 (32.5) |
| | Black | 10 (2.6) | 12 (3.1) |
| | Other | 93 (24.2) | 100 (26.0) |
| | Non-Hispanic | 210 (54.5) | 221 (57.4) |
| | Hispanic | 175 (45.5) | 164 (42.6) |

| Region of enrollment, n (%) | | | |
|---|---|---|---|
| | Central and South America | 176 (45.7) | 165 (42.9) |
| | Central and East Asia | 151 (39.2) | 144 (37.4) |
| | Eastern Europe and Russia | 49 (12.7) | 57 (14.8) |
| | United States | 8 (2.1) | 18 (4.7) |
| | South Africa | 1 (0.3) | 1 (0.3) |

| ECOG performance status score, n (%) | | | |
|---|---|---|---|
| | 0 | 265 (68.8) | 255 (66.2) |
| | 1 | 119 (30.9) | 130 (33.8) |
| | 2 | 1 (0.3) | 0 |

| Histologic type, n (%) | | | |
|---|---|---|---|
| | Squamous carcinoma | 322 (83.6) | 320 (83.1) |
| | Adenocarcinoma | 55 (14.3) | 58 (15.1) |
| | Adenosquamous carcinoma | 8 (2.1) | 7 (1.8) |

| FIGO 2009 stage, n (%) | | | |
|---|---|---|---|
| | IB2 | 19 (4.9) | 20 (5.2) |
| | IIA | 21 (5.5) | 13 (3.4) |
| | IIB | 95 (24.7) | 97 (25.2) |
| | IIIA | 54 (14.0) | 64 (16.6) |
| | IIIB | 171 (44.4) | 172 (44.7) |
| | IVA | 25 (6.5) | 19 (4.9) |

| Nodal involvement, n (%) | | | |
|---|---|---|---|
| | N0 | 106 (27.5) | 94 (24.4) |
| | N1 | 279 (72.5) | 291 (75.6) |
| Data are n (%). ECOG=Eastern Cooperative Oncology Group. FIGO=International Federation of Gynecology and Obstetrics. IQR=interquartile range. | | | |

**Table 11. PFS benefit in Durva + CRT versus Placebo + CRT increased with increasing PD-L1 cut-point.**

| **Subgroup** | | **Treatment group** | **N** | **Number (%) of subjects with events** | **Median** | **Hazard Ratio** | **95% CI** |
|---|---|---|---|---|---|---|---|
| | | | | | | | |
| PD-L1 expression (20%) | <20% | Durva + SoC CCRT | 180 | 59 (32.8) | NC | 1.06 | 0.74, 1.53 |
| | | Placebo + SoC CCRT | 186 | 57 (30.6) | NC | | |
| | >=20% | Durva + SoC CCRT | 191 | 45 (23.6) | NC | 0.62 | 0.42, 0.91 |
| | | Placebo + SoC CCRT | 178 | 63 ( 35.4) | NC | | |
| | missing | Durva + SoC CCRT | 14 | 8 ( 57.1) | 13.0 | NC | NC, NC |
| | | Placebo + SoC CCRT | 21 | 8 ( 38.1) | NC | | |
| | | | | | | | |
| PD-L1 expression (30%) | <30% | Durva + SoC CCRT | 218 | 69 ( 31.7) | NC | 0.98 | 0.70, 1.37 |
| | | Placebo + SoC CCRT | 219 | 69 (31.5) | NC | | |
| | >=30% | Durva + SoC CCRT | 153 | 35 ( 22.9) | NC | 0.62 | 0.40, 0.95 |
| | | Placebo + SoC CCRT | 145 | 51 ( 35.2) | NC | | |
| | missing | Durva + SoC CCRT | 14 | 8 ( 57.1) | 13.0 | NC | NC, NC |
| | | Placebo + SoC CCRT | 21 | 8 ( 38.1) | NC | | |
| | | | | | | | |
| PD-L1 expression (40%) | <40% | Durva + SoC CCRT | 254 | 74 (29.1) | NC | 0.92 | 0.67, 1.27 |
| | | Placebo + SoC CCRT | 248 | 76 ( 30.6) | NC | | |
| | >=40% | Durva + SoC CCRT | 117 | 30 ( 25.6) | NC | 0.65 | 0.40, 1.02 |
| | | Placebo + SoC CCRT | 116 | 44 ( 37.9) | NC | | |
| | missing | Durva + SoC CCRT | 14 | 8 ( 57.1) | 13.0 | NC | NC, NC |
| | | Placebo + SoC CCRT | 21 | 8 ( 38.1) | NC | | |
| PD-L1 expression (50%) | <50% | Durva + SoC CCRT | 276 | 82 ( 29.7) | NC | 0.95 | 0.70, 1.29 |
| | | Placebo + SoC CCRT | 270 | 82 ( 30.4) | NC | | |
| | >=50% | Durva + SoC CCRT | 95 | 22 ( 23.2) | NC | 0.54 | 0.31,0.90 |
| | | Placebo + SoC CCRT | 94 | 38 ( 40.4) | NC | | |
| | missing | Durva + SoC CCRT | 14 | 8 ( 57.1) | 13.0 | NC | NC, NC |
| | | Placebo + SoC CCRT | 21 | 8 ( 38.1) | NC | | |
| | | | | | | | |
| PD-L1 expression (60%) | <60% | Durva + SoC CCRT | 289 | 87 ( 30.1) | NC | 0.97 | 0.72, 1.31 |
| | | Placebo + SoC CCRT | 284 | 86 ( 30.3) | NC | | |
| | >=60% | Durva + SoC CCRT | 82 | 17 (20.7) | NC | 0.44 | 0.24, 0.77 |
| | | Placebo + SoC CCRT | 80 | 34 ( 42.5) | NC | | |
| | missing | Durva + SoC CCRT | 14 | 8 ( 57.1) | 13.0 | NC | NC, NC |
| | | Placebo + SoC CCRT | 21 | 8 ( 38.1) | NC | | |
| For each subgroup the analyses are performed using a Cox proportional hazards model that contains only a term for treatment using Efron method to control for ties. | | | | | | | |
| A hazard ratio <1 implies a lower risk of progression on Durva + SoC CCRT. Progression includes deaths in the absence of progression. | | | | | | | |
| Progression events that occur after 2 or more consecutive missed visits following last evaluable assessment are censored. Region recorded on eCRF. | | | | | | | |
| If there are less than 20 events in the subgroup, only descriptive summaries are provided without HR and 95% CI of HR. | | | | | | | |
| Durva = Durvalumab; SoC = Standard of Care; CCRT = Concurrent Chemoradiation Therapy; HR = Hazard Ratio RECIST version 1.1 | | | | | | | |

### Example 2: A Phase 1, Open-label, Dose-escalation and Dose-expansion Study to Evaluate the Safety, Tolerability Pharmacokinetics Immunogenicity, and Antitumor Activity of MEDI5752 in Subjects with Advanced Solid Tumors

MEDI5752 was assessed in a Phase 1, first-time-in-human, multicenter, open-label, dose-escalation and dose-expansion study to evaluate the safety and tolerability and early evidence of efficacy of MEDI5752 in adult subjects with advanced solid tumors when administered as a single agent or combined with chemotherapy. The purpose of this study was to provide the safety profile, description of pharmacokinetics (PK), pharmacodynamics (PD), and early signs of antitumor efficacy. Subjects were to be enrolled at approximately 50 sites globally. As of the data cut-off date, a total of 178 participants with advanced solid tumors received at least one dose of MEDI5752 monotherapy at one of 10 dose levels (1 each at 2.25 mg and 7.5 mg, 3 at 22.5 mg, 5 at 75 mg, 10 at 225 mg, 20 at 500 mg, 40 at 750 mg, 39 at 1500 mg, 34 at 2000 mg, and 7 at 2500 mg). The study included 2 phases: dose escalation and dose expansion (Figs. 1A-1D). The dose escalation phase evaluated 10 dose levels to identify a maximum tolerated dose (MTD), optimal biological dose (OBD), or highest protocol-defined dose (HPDD) dose.

Dose escalation consisted of 10 dose levels of MEDI5752 (dose levels 2.25, 7.5, 22.5, 75, 225, 500, 750, 1500, 2000, and 2500 mg) administered via IV infusion. Once the OBD, MTD or HPDD was determined, a specific cohort of up to 6 subjects with a glomerular filtration rate (GFR) between 30 and 45 mL/min could be explored at the OBD, MTD, or HPDD (ie, pharmacodynamic cohort). Up to approximately 111 subjects were to be enrolled in the dose-escalation phase.

The dose-expansion phase was initiated once the MTD, OBD, or HPDD was established in the dose-escalation phase. In the expansion cohorts, only immunotherapy-naïve eligible metastatic subjects were enrolled.

The primary safety endpoints for the dose-escalation phase were the assessment of the presence of AEs, SAEs, and DLTs and the determination of the MTD, OBD, or HPDD of MEDI5752 in the absence of exceeding the MTD.

A total of 136 subjects had been treated with MEDI5752. Table 5 shows the exposure and the MEDI5752-related AEs observed in selected MEDI5752 monotherapy and combination dose levels as of the data snapshot date.

**Table 5 MEDI5752-related Adverse Events as of 25Jan2023 (Data Snapshot Date)**

| **Subjects with at least one:** | **Monotherapy** | | | | | **Combination with:** | | | |
|---|---|---|---|---|---|---|---|---|---|
| | **All N=178** | **225 mg N=10** | **500 mg N=38** | **750 mg N=40** | **1500 mg N = 39** | **Carboplatin and Pemetrexed** | | | **Carbop latin and Taxane** |
| | | | | | | **500 mg N=33** | **750 mg N=72** | **1500 mg N=20** | **750 mg N=18** |
| Median Duration of Exposure (months) | 3.50 | 3.12 | 5.42 | 3.79 | 3.48 | 2.99 | 4.14 | 4.16 | 2.00 |
| Range of Duration of Exposure (months) | 0.5, 50.4 | 1.3, 50.4 | 0.7, 23.5 | 0.5, 18.6 | 0.5, 14.0 | 0.6, 6.2 | 0.3, 16.4 | 0.7, 25.0 | 0.7, 7.6 |
| Median Number of Cycles | 4.00 | 4.00 | 7.50 | 5.00 | 4.00 | 4.00 | 5.50 | 4.50 | 3.00 |
| Range of Cycles | 1.0, 71.0 | 2.0, 71.0 | 1.0, 33.0 | 1.0, 27.0 | 1.0, 19.0 | 1.0, 9.0 | 1.0, 17.0 | 1.0, 33.0 | 1.0, 10.0 |
| AEs (All Grades) | 89.3% | 70% | 86.8% | 95% | 94.9% | 72.7% | 80.6% | 100% | 77.8% |
| Grade 3 or 4 AEs | 46.1% | 10% | 31.6% | 55% | 59% | 18.2% | 36.1% | 70% | 44.4% |
| SAEs | 34.8% | 20% | 18.4% | 45% | 43.6% | 18.2% | 23.6% | 55% | 16.7% |

Dose-limiting toxicities (DLTs) were reported in one subject each in the 2000 mg (Grade 3 pneumonitis and Grade 1 myocarditis in the same subject) and 2500 mg (Grade 3 maculopapular rash) monotherapy cohorts. Two deaths were judged to be related to MEDI5752 treatment; one subject died due to diabetic ketoacidosis and hyperthyroidism and another subject died due to myocardial infarction.

Review of the benefit-risk profile of subjects treated with MEDI5752 suggested that long-term dosing at 1500 mg and above were not suitable for further development due to high treatment discontinuation rates, primarily driven by Grade 3/4 hepatotoxicity.

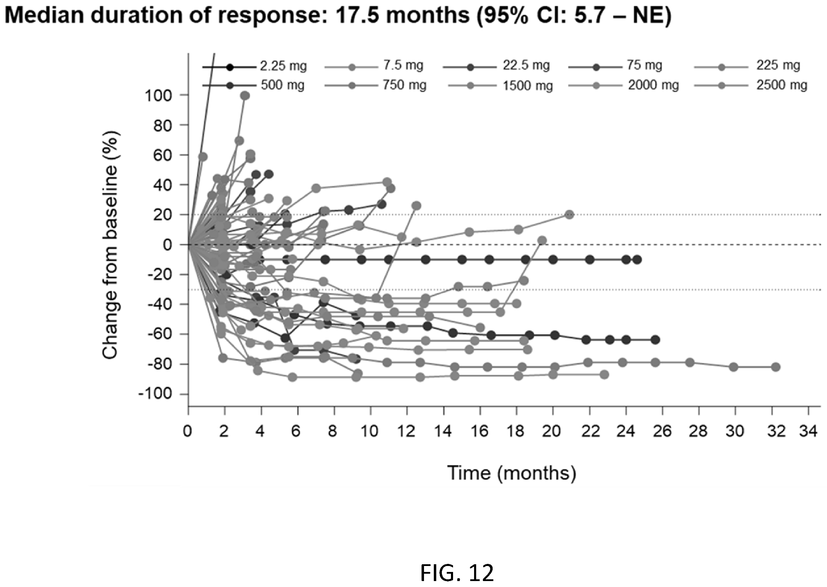

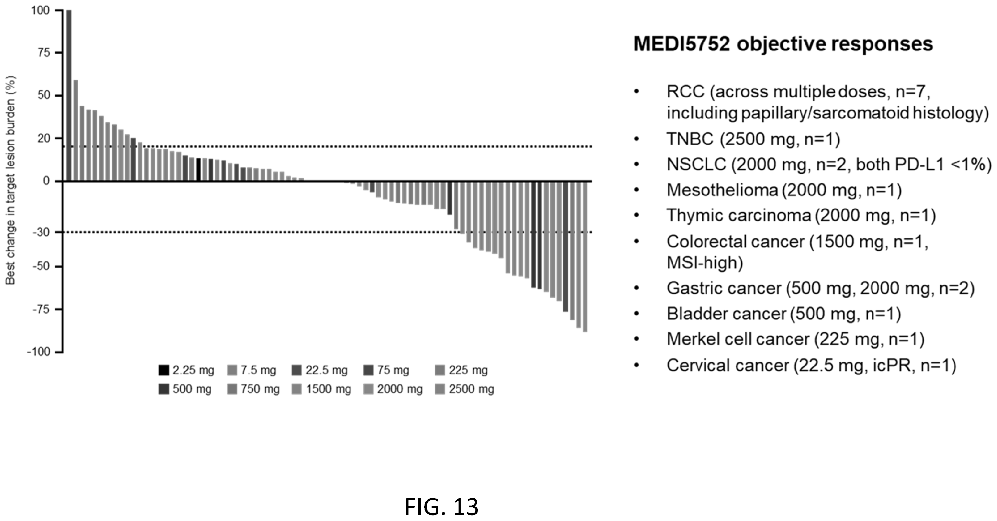

Among 86 MEDI5752 monotherapy subjects in the dose-escalation and maximum tolerated dose (MTD)/optimal biological dose (OBD) cohorts, regardless of dose, in the response evaluable analysis set, the overall objective response rate (ORR) (per Response Evaluation Criteria in Solid Tumors [RECIST] version [v]1.1) was 19.8% (17/86) with 1 complete response (CR). In the 5 subjects treated with 500 mg of MEDI5752 during dose escalation, 2 subjects had a best overall response (BOR) of partial response (PR), 2 subjects had stable disease (SD), and 1 subject had progressive disease (PD). In the 8 subjects treated with 750 mg of MEDI5752 during dose escalation, 4 subjects had a BOR of SD, 3 had PD, and 1 subject was not evaluable. The median duration of response for MEDI5752 monotherapy was 17.5 months (FIG. 12). MEDI5752 monotherapy also showed a durable response in diverse IO-naïve tumors across a range of MEDI5752 doses (FIG. 13).

In this first time in human trial, three subjects with relapsed/refractory cervical cancer were treated with MEDI5752 (one at 22.5 mg, one at 1500 mg, and one at 2000 mg) in the dose escalation phase. All three subjects had a best observed response of stable disease.

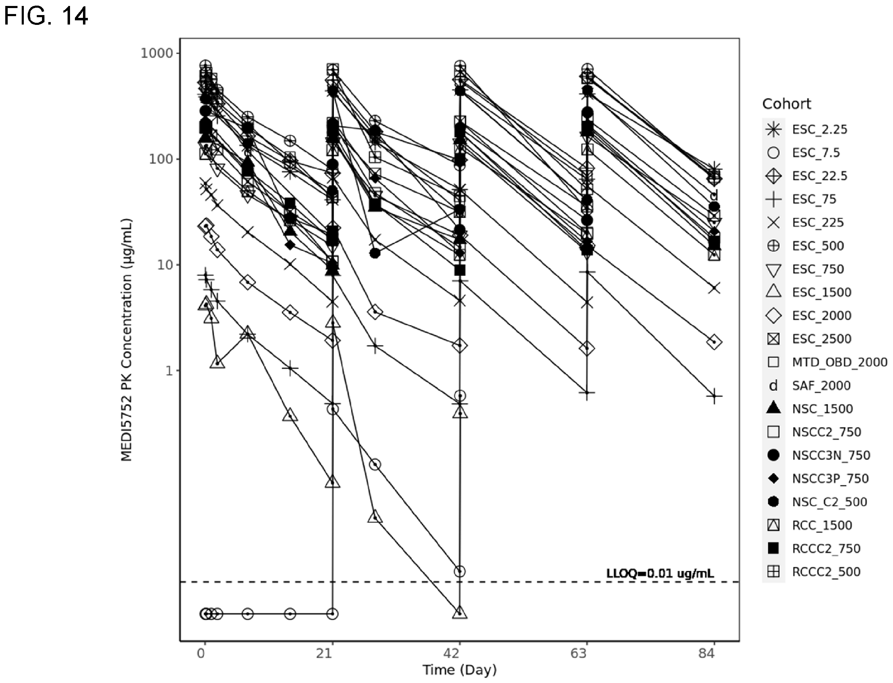

Data suggested that MEDI5752 exhibits nonlinear PK likely due to saturable target-mediated clearance at doses < 22.5 mg and additionally likely potentially due to the impact of ADA on clearance of MEDI5752. Mean MEDI5752 PK profiles over the first 84 days are shown in FIG. 14.

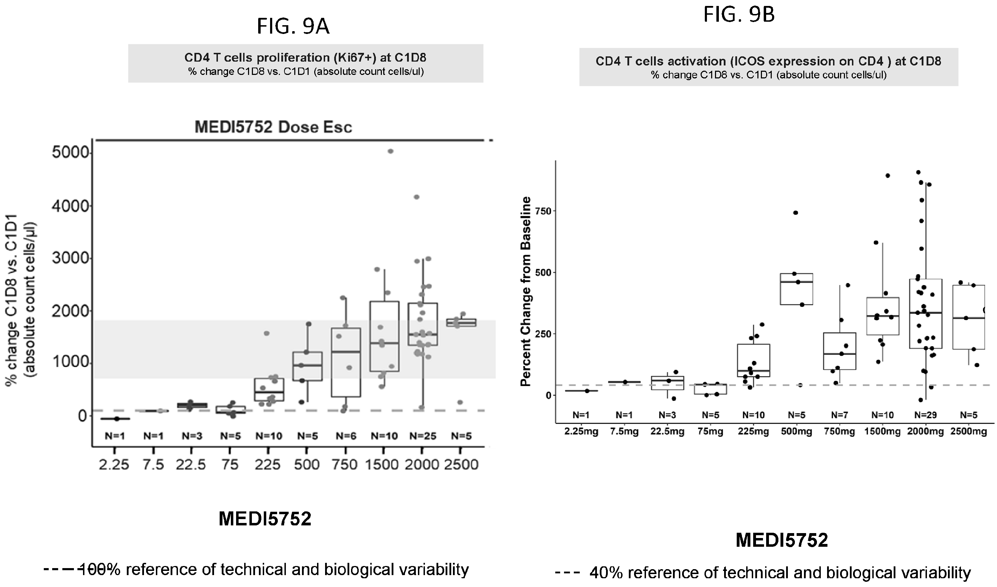

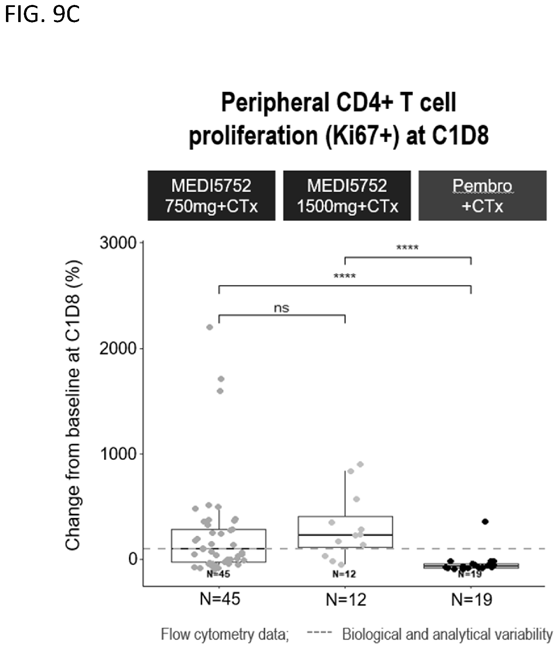

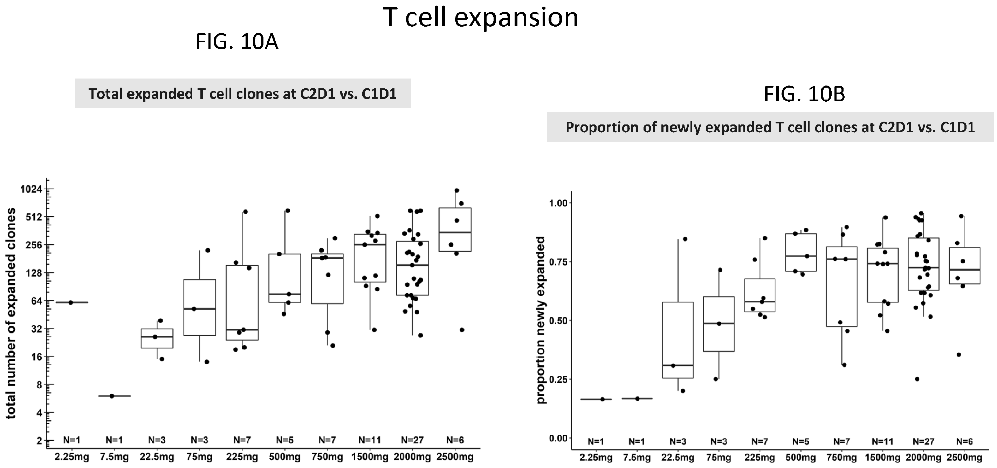

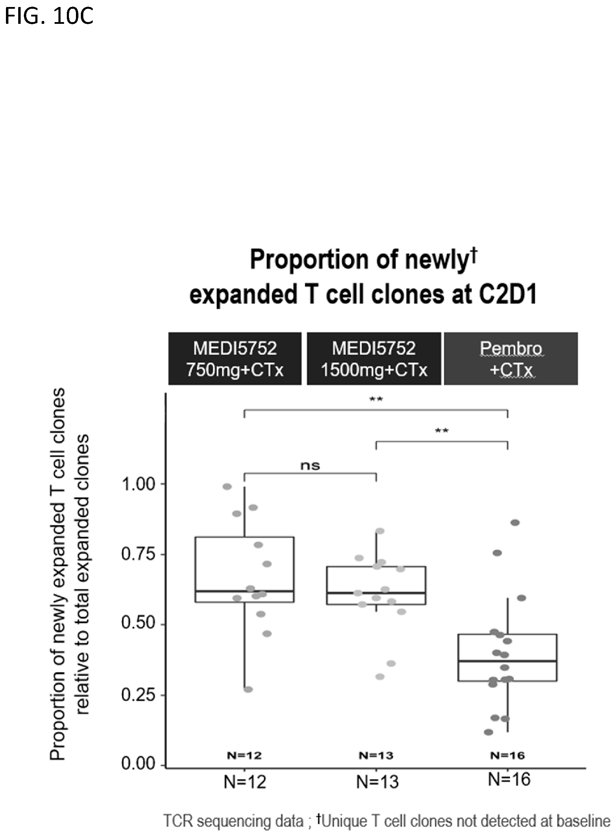

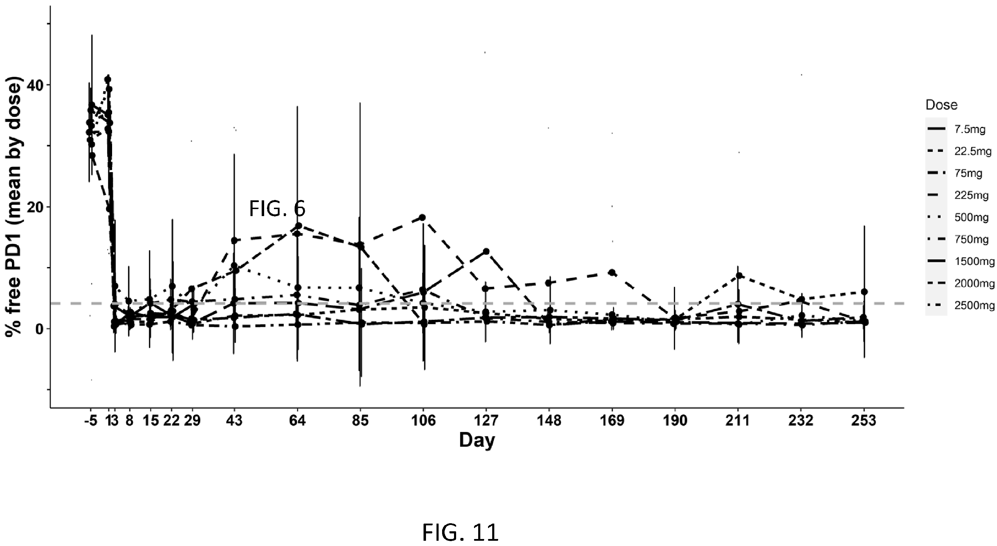

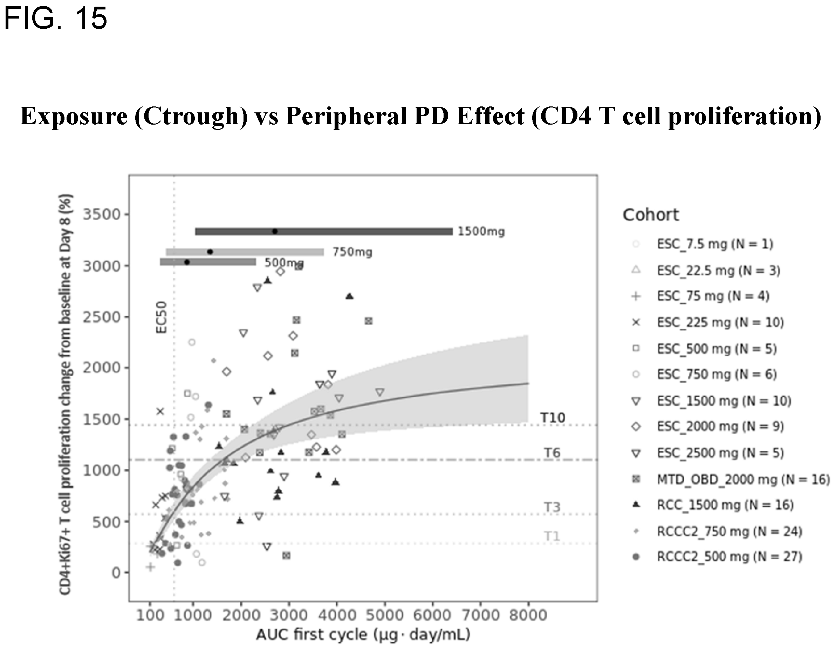

Pharmacodynamic data suggested MEDI5752 administration leads to a dose-dependent increase in the CD4+ T cell proliferation, plateauing at 500/750 mg and above (FIG. 15 and FIG. 9A). At doses of >225 mg, MEDI5752 demonstrated sustained peripheral PD-1 receptor occupancy (>90%) (FIG. 11). Pharmacodynamic data in monotherapy setting also suggested MEDI5752 leads to a dose-dependent increase in the CD4+ T cell activation, and T cell expansion plateauing at 500/750 mg and above in (FIG. 9B and FIGs. 10A-B respectively). Pharmacodynamic data in chemotherapy combination setting showed a higher increase in the CD4+ T cell proliferation at cycle 1 day 8 and higher proportion of newly expanded T cell clones at cycle 2 at doses of 750mg and 1500mg MEDI5752 in combination with chemometherapy in comparison to pembrolizumab in combination with chemotherapy (FIG. 9C and 10C respectively).

### Example 3: Efficacy of Volrustomig (MEDI5752) in Patients with Cervical Cancer

There remains a significant unmet need for additional treatment options for patients with cervical cancer. This is a phase III, randomized, double-blind, placebo-controlled, multi-center, international study to explore the efficacy and safety of volrustomig in women with FIGO (2018) high risk LACC who have not progressed following platinum-based CCRT.

The study design aims to minimize potential risks to participants participating in this study based on the protocol inclusion and exclusion criteria, safety monitoring, TMGs and study stopping criteria. Specific intensive safety monitoring is in place.

Volrustomig is being evaluated in a patient population with limited life expectancy due to lymph node metastasis. While there are available treatment options for such patients, volrustomig has the potential to offer an improved benefit-risk profile. Based on the review of available safety data for volrustomig, the benefit-risk profile of volrustomig remains acceptable for continued investigation and is justified by the perceived benefits for patients with cancer.

**Table 12. Study objectives and Endpoints.**

| **Objectives** | **Endpoints** | |
|---|---|---|
| **Primary** | | |
| To assess the efficacy of volrustomig compared with placebo in LACC participants post SoC CCRT in terms of PFS by investigator in PD-L1 high expression population. | Endpoint based on the investigator assessment according to RECIST 1.1 or histopathologic confirmation of local tumor progression: | |
| | | PFS: Time from date of randomization until tumor progression or death due to any cause regardless of whether the participant withdraws from therapy, receives another anti-cancer therapy or clinically progresses prior to RECIST 1.1 progression. However, if the participant progresses or dies immediately after 2 or more consecutive missed visits, the participant is censored at the time of the last date of assessment which the RECIST 1.1 assessment |

| **Secondary** | | |
|---|---|---|
| To assess the efficacy of volrustomig compared with placebo in terms of PFS by investigator in ITT population. | As per primary endpoint. | |
| To assess the efficacy of volrustomig compared with placebo in terms of OS in PD-L1 high expression/ITT population. | OS: Time from randomization until the date of death due to any cause. | |
| | The comparison includes all randomized participants in the specific population, regardless of whether the participant withdraws from therapy or receives another anti-cancer therapy. | |
| To assess the efficacy of volrustomig compared with placebo in terms of ORR. | ORR: The percentage of evaluable participants with an Investigator-assessed visit response of CR or PR. | |
| To assess the efficacy of volrustomig compared with placebo in terms of DoR in participants with a CR or PR. | DoR in participants with a CR or PR: Time from date of first detection of CR or PR until the date of objective disease progression according to RECIST 1.1. | |
| To assess the efficacy of volrustomig compared with placebo in terms of PFS at 12 months in PD-L1 high expression/ITT population. | PFS rate at 12 months: The Kaplan-Meier estimate of PFS at Month 12. | |
| To assess the efficacy of volrustomig compared with placebo in terms of PFS at 24 months in PD-L1 high expression/ITT population. | PFS rate at 24 months: The Kaplan-Meier estimate of PFS at Month 24. | |
| To assess the efficacy of volrustomig compared with placebo in terms of OS at 36 months in PD-L1 high expression or ITT population. | OS rate at 36 months: The Kaplan-Meier estimate of OS at Month 36. | |
| To assess the efficacy of volrustomig compared with placebo in terms of TFST in PD-L1 high expression or ITT population. | TFST: The time from randomization until the start date of the first subsequent anti-cancer therapy after discontinuation of randomized treatment, or death due to any cause. | |
| To assess the efficacy of volrustomig compared with placebo in terms of PFS2 in PD-L1 high expression or ITT population. | PFS2: The time from randomization to the earliest of the progression event (following the initial investigator-assessed progression), after first subsequent therapy, or death. The date of second progression is recorded by the investigator in the eCRF and defined according to local standard clinical practice. | |
| To assess the efficacy of volrustomig compared with placebo in terms of PFS by BICR in PD-L1 high expression or ITT population. | Endpoints based on the BICR assessment according to RECIST 1.1: PFS | |
| To assess the effect of volrustomig compared with placebo on the incidence of local progression, distant disease progression, and secondary malignancy as the first documented progression event. | Incidence of Local Progression, Distant Disease Progression, and Secondary Malignancy: Number and percentage of participants who develop local progression, distant disease recurrence, or secondary malignancy. | |
| To assess the PK of volrustomig. | Concentration of volrustomig in serum and PK parameters parameters as data allow. | |
| To investigate the immunogenicity of volrustomig. | Incidence of ADAs against volrustomig in serum. | |
| To assess the safety and tolerability profile of volrustomig compared to placebo. | AEs, laboratory findings, vital signs, and physical examinations. | |
| To describe participant-reported symptomatic AEs and treatment tolerability. | Proportion of participants experiencing different levels of treatment-related symptoms as measured by selected items from the PRO-CTCAE and EORTC and reporting different levels of overall tolerability as measured by the PGI-TT. | |
| To assess participant-reported symptoms, functioning, and HRQoL. | EORTC IL172 (QLQ-C30 functioning subscales + HRQoL items) EORTC IL: QLQ-CX24 PROMIS Physical Function 8c | |
| To assess participant-reported global impression of severity and change of overall cancer symptoms. | Proportion of participants reporting different levels of global impression of the severity of overall cancer symptoms as measured by PGI-S. | |
| To assess participant-reported health-related quality of life. | VAS score, its change from baseline and 5-dimension scores as measured by EQ-5D-5L. | |

| **Exploratory** | | |
|---|---|---|
| To assess peripheral and intra-tumoral changes induced by sequential CRT and volrustomig treatment as pharmacodynamics biomarkers. | Assessment of changes in tumor and immune-associated marker profiles in peripheral blood and tumor tissue biopsies by DNA, RNA, or protein measurements, including but not limited to ctDNA, gene expression profiling, TCR clonality changes. | |
| To evaluate the association of tumor baseline characteristics with the anti-tumor activity of sequential CRT and volrustomig treatment as predictive biomarkers. | Assessment of baseline tumor and immune-associated markers in peripheral blood and tumor tissue biopsies by DNA, RNA, or protein measurements, including but not limited to PD-L1 expression level, DNA mutation profiles, RNA expression profiles, immune cell infiltration status. | |
| Abbreviations: ADA=antidrug antibody; AE=adverse event; BICR=blinded independent central review; CCRT=concurrent chemoradiation therapy; CR=complete response; CRT=chemoradiation therapy; ctDNA=circulating tumor DNA; DNA=deoxyribonucleic acid; DoR=duration of response; eCRF=electronic case report form; EORTC=European Organization for Research and Treatment of Cancer; EQ-5D-5L=EuroQol five-dimensional five-level questionnaire; HRQoL=health-related quality of life; IL=item library; ITT=intent to treat; LACC=locally advanced cervical cancer; ORR=overall response rate; OS=overall survival; PD-L1=programmed death-ligand 1; PFS=progression-free survival; PFS2=time to second progression or death; PGI-C=Patient's Global Impression of Change; PGI-S=Patient's Global Impression of Severity; PGI-TT=Patient's Global Impression of Treatment Tolerability; PK=pharmacokinetics; PR=partial response; PRO-CTCAE=patient-reported outcomes version of the Common Terminology Criteria for Adverse Events; QLQ-C30=30-item core quality of life questionnaire; QLQ-CX24=cervical cancer module; RECIST 1.1=Response Evaluation Criteria in Solid Tumors, Version 1.1; RNA=ribonucleic acid; TFST=time to first subsequent therapy or death; SoC=standard of care; TMB=tumor mutational burden; TCR=T-cell receptor; VAS=visual analogue scale | | |

### STUDY DESIGN

Approximately 1430 participants are screened, in order to randomize 1000 participants in a 1:1 ratio to two arms. Randomization is stratified by PD-L1 expression as assessed by central laboratories using the VENTANA PD-L1 (SP263) Assay (PD-L1 high expression vs others), as well as Regions (Asia vs non-Asia).
- Arm A: Volrustomig 750 mg IV on Day 1 of each 21-day cycle until disease progression or up to 24 months (n = 500).
- Arm B: Placebo 750 mg IV on Day 1 of each 21-day cycle until disease progression or up to 24 months (n = 500).

Participants receive their assigned treatment up to 24 months, or until clinical or RECIST 1.1-defined radiological progression, unacceptable toxicity, withdrawal of consent, or another discontinuation criterion is met. After 24 months, participants may continue treatment if in the investigator's opinion, they are benefiting from treatment and do not meet any other discontinuation criteria. Participants who discontinue for reasons other than radiographic disease progression will have post-treatment follow-up imaging for disease status until radiographic disease progression, withdrawing consent, becoming lost to follow-up or death.

### Scientific Rationale for Study Design

This is a phase III, randomized, double-blind, placebo-controlled, multi-center, international study to explore the efficacy and safety of volrustomig in women with FIGO (2018) high risk LACC who have not progressed following platinum-based CCRT. Volrustomig, a monovalent bispecific humanized IgG1 mAb that specifically binds to CTLA-4 and PD-1, is administered post SoC CCRT and compared to placebo post SOC CCRT in participants with high risk LACC.

Concurrent chemoradiation therapy has been shown to induce immunogenic cell death. Cell death, from radiation or CCRT, enhances the ability of the immune system to recognize and respond to the tumor through enhanced antigen release and presentation. In addition, ionizing radiation causes upregulation of various pro-inflammatory signals and cytokines, which play a key role in immune regulatory pathways, leading to improved anti-tumor immunity. Radiation also enhances the diversity of the T-cell receptor repertoire of intra-tumoral T cells.

Cytotoxic T-lymphocyte antigen 4 is a negative regulator of T cell activation and its inhibition allows T cell response against cancer. Likewise, the PD-1 axis is involved in the negative T cell regulation and its inhibition leads to the recovery of the cytotoxicity of T cells towards tumors. Recent studies showed that tumor-specific CD8+ T cells express several inhibitory receptors (including CTLA-4, P-1, TIM-3, BTLA, and LAG3) and their double simultaneous blockade acts synergistically to render T cells more functional than a single blockade (Huang *et al*. 2015).

### STUDY POPULATION

The target population of interest in this study is participants with FIGO (2018) Stage IIIC to IVA cervical cancer (with lymph node positive status), who have not progressed on definitive, platinum-based CCRT. Prospective approval of protocol deviations to recruitment and enrolment criteria, also known as protocol waivers or exemptions, is not permitted. Participants who do not meet the eligibility criteria requirements are screen failures.

### Inclusion Criteria

Participants are eligible to be included in the study only if all of the following criteria apply:
Age
1 Participant must be ≥ 18 years at the time of screening. For sites which plan to enroll pediatric participants, ≥ 15 years at the time of screening.
   Type **of Participant and** Disease Characteristics
2 Participants must have histologically documented FIGO (2018) Stages IIIC to IVA cervical cancer (with lymph node positive status). Participants must have histologically confirmed cervical adenocarcinoma, cervical squamous carcinoma, or cervical adenosquamous carcinoma and the following requirements:
   - Only participants with LN involvement are included.
   - Nodal involvement confirmation may be either surgical or by imaging (PET-CT, CT or MRI) with pathological lymph node size defined by a short-axis diameter of ≥ 10 mm (axial plane)
   - No evidence of metastatic disease (M0)
3 Initial staging procedures performed prior to initiation of any component of definitive treatment (CCRT) should include:
   - Pelvic MRI (preferred) or CT with IV contrast; Chest/abdomen CT with IV contrast. PET-CT is recommended in addition but not mandatory. Brain MRI (preferred) or CT with IV contrast only if symptomatic.
   - The above scans for initial staging procedures must have been performed up to 42 days prior to the first dose of CCRT.
4 WHO/ECOG performance status of 0 or 1 at enrolment and randomization.
5 Life expectancy ≥ 12 weeks.
6 Provision of FFPE tumor sample to assess the PD-L1 expression as determined by a central laboratory using the VENTANA PD-L1 (SP263) Assay prior to randomization. Patients with unknown PD-L1 expression are not eligible for study.
   - Tumor sample requirements as follows: FFPE sample must be collected less than 3 months old prior to CCRT.
7 Participants must not have progressed following definitive, platinum-based, CCRT as demonstrated by the following imaging studies performed after completion of CCRT. Screening baseline RECIST 1.1 imaging of chest/abdomen by CT with IV contrast, pelvic MRI (preferred) or CT with IV contrast.
**8** Must have completed SOC CCRT (EBRT + brachytherapy + concurrent cisplatin) per local practice/guideline and must not have progressed following CCRT. Participants are strongly preferred to complete CCRT within 8 weeks. Participants must have received weekly cisplatin 40 mg/m² for 5-6 cycles as concurrent chemotherapy with radiation therapy, which must be completed within 1 to 28 days prior to first dose of study intervention in the study (one chemotherapy cycle is defined as 7 days). If participants cannot tolerate toxicity, participants must have received at least 4 cycles of cisplatin. Participants need to complete screening within 28 days post CCRT. The platinum chemotherapy regimen must be cisplatin monotherapy, the last dose of chemotherapy must be administered prior to, or concurrently with, the final dose of radiation. Consolidation chemotherapy after radiation is not permitted. Adequate bone marrow reserve and organ function within 7 days before randomization/treatment

**Dose level**: A fixed dose of 500 mg or 750 mg of volrustomig (MEDI5752) administered Q3W.

**Route of administration**: IV

**Regimen and duration**: One IV infusion Q3W on Day 1 of each 21-day cycle. Administered as an IV infusion over 1 hour (± 10 minutes).

**Duration of Treatment**: Participants in both the volrustomig 750 mg and 500 mg group and the placebo group receive their assigned treatment up to 24 months, or until clinical or RECIST 1.1-defined radiological progression, unacceptable toxicity, withdrawal of consent, or another discontinuation criterion is met. After 24 months, participants may continue treatment with volrustomig if in the investigator's opinion they are benefiting from treatment and do not meet any other discontinuation criteria.

### Efficacy Assessments

### Imaging Tumor Assessments

Tumor assessments use images from pelvic MRI (preferred) or CT with IV contrast and chest/abdomen (including the entire liver and both adrenal glands) CT with IV contrast (PET-CT is recommended in addition but not mandatory), collected during screening/baseline and at regular (follow-up) intervals during the Intervention Period. Any other areas of disease involvement should be additionally imaged at screening based on known metastasis sites or by the signs and symptoms of individual participants. The imaging modality used for baseline tumor assessments (pelvic MRI [preferred] or CT with IV contrast and chest/abdomen CT with IV contrast [pelvic and chest/abdomen PET-CT is recommended in addition but not mandatory]; brain MRI [preferred]) or CT with IV contrast only if symptomatic) should be kept the same consistently at each subsequent follow-up assessment throughout the study if possible. It is important to follow the tumor assessment schedule as closely as possible (refer to the SoA) relative to randomization/first dose.

Screening/baseline imaging results must be obtained within 28 days of randomization and ideally should be performed as close as possible to and prior to the start of study intervention. Study treatment continues until completion of planned therapy (up to 24 months), clinical or RECIST 1.1-defined radiological progression, unacceptable toxicity, withdrawal of consent, or another discontinuation criterion is met.

Scanning/tumor assessments is performed every 12 weeks ± 1 week through 158 weeks (relative to the date of randomization) and then every 24 weeks ± 2 weeks thereafter (relative to the date of randomization) until RECIST 1.1-defined radiological progression plus an additional follow-up scan. Participants who discontinue for reasons other than radiographic disease progression have post-treatment follow-up imaging for disease status until radiographic disease progression or, withdrawing consent, or becoming lost to follow-up or death.

If an unscheduled assessment is performed (*e.g*., to investigate clinical signs/symptoms of progression) and the participant has not progressed, every attempt should be made to perform the subsequent assessments at the next scheduled visit.

The RECIST 1.1 assessments of baseline images identify TLs (defined as measurable) and NTLs. On-study images are evaluated for TLs and NTLs chosen at baseline, and for NLs when they appear. This allows determination of follow-up TL response, NTL lesion response, the presence of unequivocal NLs, and overall time point responses (CR, PR, SD, disease progression, or NE).

### Central Reading of Scans

Images, including unscheduled visit scans, are collected on an ongoing basis and sent to an AstraZeneca-appointed iCRO for quality control, and storage. Digital copies of all original scans should be stored at the investigator site as source documents. Electronic image transfer from the sites to the iCRO is strongly encouraged. A BICR of images is performed at the discretion of AstraZeneca. Results of these independent reviews are not communicated to investigators, and results of investigator tumor assessments are not shared with the central reviewers.

### Time to Second Progression or Death

Following first objective progression participants have their subsequent progression status recorded every 12 weeks according to local standard practice by the investigator until the second progression event has occurred after the start of a subsequent anti-cancer therapy.

Assessments Prior to Start of Subsequent Anti-cancer Therapy: If at the subsequent progression assessment, the subsequent anti-cancer therapy has not started, subsequent progression is assessed in relation to the first objective RECIST 1.1 progression. In addition, the reasons for not starting a subsequent anti-cancer therapy are recorded.

Assessments After Start of Subsequent Anti-cancer Therapy: If at the PFS2 assessment, the subsequent anti-cancer therapy has started, then second progression is assessed in relation to the last tumor assessment prior to starting the subsequent anti-cancer therapy.

The investigator opinion of progression status (progressive disease, nonprogressive disease) is recorded, and if progressive disease is recorded, then the type of progression is also recorded.

### Overall Survival

Assessments for survival are conducted every 12 weeks following the 30- and 90-day post-treatment discontinuation safety follow-up visits. Survival information may be obtained via telephone contact with the participant, participant's family, by contact with the participant's current physician, or local death registries. Note: Survival calls are made following the data cut-off (DCO) date for the analysis (these contacts should generally occur within 7 days of the DCO date). If participants are confirmed to be alive or if the death date is after the DCO date, then these participants are censored at the date of DCO.

### Clinical Outcome Assessments (COA)

A COA is any assessment that may be influenced by human choices, judgment, or motivation and may support either direct or indirect evidence of treatment benefit. Patient-reported outcome assessment is one type of COA and is a general term referring to all outcomes and symptoms that are directly reported by the participant. Patient-reported outcomes have become important in evaluating the efficacy and tolerability of study interventions in clinical studies and aid in understanding of the benefit/risk evaluation (Kluetz *et al.* 2018). The following PRO instruments are administered in this study:
- EORTC IL172 (QLQ-C30 functioning subscales + HRQoL items)
- EORTC IL: QLQ-CX24
- EQ-5D-5L
- PGI-C
- PGI-S
- PGI-TT
- PRO-CTCAE
- PROMIS Physical Function 8c

Patient-reported outcomes instruments are administered according to the SoA. The PRO instruments are completed by participants if a linguistically validated version is available in their language for the country in which they reside.

### Sample Size Determination

Approximately 1430 participants are enrolled to achieve approximately 1000 randomized participants to volrustomig or placebo in a 1:1 ratio. Out of the approximately 1000 participants randomized into the study, approximately 500 participants are included in the PD-L1 high expression population analysis set. The primary analysis of PFS is planned to be performed in the PD-L1 high expression population analysis set. An analysis of PFS in the FAS is performed as a secondary objective of the study.

The primary (final) PFS analysis for superiority is performed when approximately 162 PFS events have occurred across the volrustomig and placebo treatment arms (approximately 32% maturity) in the PD-L1 high expression population analysis set. If the true PFS HR is 0.60 in this population, the analysis provides 90% power to demonstrate a statistically significant difference for PFS with a 2-sided significance level of 5%; this translates to an approximately 14% increase in the proportion of participants with progression-free at 2 years from 58% in placebo to 72% if PFS is exponentially distributed. It is estimated that this analysis occurs approximately 40 months following randomization of the first participant.

The analysis of PFS in the FAS is performed at the time of primary PFS analysis. It is estimated that approximately 342 PFS events are expected in the FAS. If the true PFS HR is 0.70 in this population, the analysis provides at least 90% power to demonstrate a statistically significant difference for PFS with a 2-sided significance level of 5%; this translates to an approximately 10% increase in the proportion of participants with PFS at 2 years from 58% in placebo to 68% if PFS is exponentially distributed.

If the true OS HR is 0.60 in the PD-L1 high expression population analysis set, the study is also sized to provide at least 90% power for the OS endpoint in the comparison of volrustomig versus placebo, assuming a 2-sided significance level allowing for 2 interim analyses conducted approximately 45% (at the time of the PFS analysis) and 75% of the target events; this translates to an approximately 10% increase in the 3-year OS rate from 72% in placebo to 82% if OS is exponentially distributed.

The interim/final analysis of OS in the FAS is performed at the time of the respective interim/final analysis of OS in the PD-L1 high expression population analysis set. It is estimated that approximately 343 OS events are expected in all randomized participants at the time of OS final analysis in the PD-L1 high expression population analysis set. If the true OS HR is 0.70 for this population, the analysis provides at least 90% power to demonstrate a statistically significant difference for OS with a 2-sided significance level allowing for 2 interim analysis conducted at the time of the 2 OS interim analysis in the PD-L1 high expression population analysis set. This translates to an approximately 7% increase in the 3-year OS rate from 72% in placebo to 82% if OS is exponentially distributed.

**Table 13. Populations for Analysis**

| **Population/Analysis set** | **Description** |
|---|---|
| Screened | All participants who sign the Main Screening ICF. |
| ITT (Randomized/Assigned to study intervention) | All participants who were randomly assigned to study intervention regardless of whether they receive treatment or not. Data from participants is analyzed according to the intervention to which they were randomly assigned. |
| PD-L1 high expression population analysis set | All participants with high expression of PD-L1 as assessed by a central reference laboratory using the VENTANA PD-L1 (SP263) Assay. |
| FAS | All participants who are randomized in the study. The FAS is used for all the efficacy analyses including PROs. Treatment groups are compared on the basis of randomized study intervention, regardless of the treatment actually received. Participants who were randomized but did not subsequently receive study intervention are included in the analysis in the treatment group to which they were randomized. |
| Safety | The Safety analysis set consists of all randomized participants who received any amount of volrustomig. |
| PK | The PK analysis set consists of all participants who received at least 1 dose of volrustomig for whom there is at least one reportable PK concentration. |
| Abbreviations: FAS=full analysis set; ICF=informed consent form; ITT=intent to treat; PD-L1=programmed death-ligand 1; PK=pharmacokinetics; PRO=patient-reported outcome | |

### Statistical Analyses

### General Considerations

The primary objective of this study is to assess the efficacy of volrustomig compared with placebo in terms of PFS as assessed by investigator tumor assessments and histopathologic confirmation of local tumor progression in the PD-L1 high expression population analysis set. The key secondary objective is to assess the efficacy of volrustomig compared with placebo in terms of PFS in the FAS, OS in the PD-L1 high expression analysis set, and OS in the FAS.

The primary endpoint of PFS is tested once. The secondary endpoint of OS is tested at two interim and at a final timepoint. The alpha level allocated to OS is controlled at the interim and final timepoints using the Lan DeMets (Lan and DeMets 1983) spending function separately for each population that approximates the O'Brien-Fleming approach, where the alpha allocated at the interim depends upon the proportion of information available at the time of analysis.

The PFS analysis is performed when PFS reaches approximately 162 events (32% maturity) in the PD-L1 high expression population analysis set. This is estimated to occur approximately 40 months after the first participant is randomized.
Overall survival in the PD-L1 high expression population analysis set is tested at two IAs and one FA as described below:
- The first OS IA is planned to be performed at the time of the final PFS analysis. It is expected that approximately 73 OS events (15% maturity or 45% information fraction) are observed in the PD-L1 high expression population analysis set.
- The second OS IA is planned to be performed when approximately 121 OS events (24% maturity or 75% information fraction) have been observed in the PD-L1 high expression population analysis set, which is expected to occur approximately 56 months after the first participant is randomized.
- The final OS analysis is performed when approximately 162 OS events have been observed (32% maturity), which is expected to occur approximately 72 months after the first participant is randomized.

The IA/FA of OS in the FAS is performed at the time of the respective IA/FA of OS in the PD-L1 high expression population analysis set.

The primary PFS analysis is based on the programmatically derived RECIST 1.1 using the investigator tumor assessments or histopathologic confirmation of local tumor progression. The PFS and OS is analyzed using a stratified log-rank test with treatment as fixed effect adjusting for randomization stratification variables. The randomization stratification variables in the statistical modelling is based on the values entered into the IRT at randomization, even if it is subsequently discovered that these values were incorrect. If there are insufficient events per stratum, the strata are pooled following a pooling strategy that is prespecified. The HR and its corresponding CI are estimated from a stratified Cox proportional hazards model with strata being the same as the randomization stratification variables from IRT.

Efficacy data are summarized and analyzed on an ITT basis, and the treatment arms are compared on the basis of randomized treatment, regardless of the treatment actually received. Patients who were randomized but did not subsequently go on to receive study treatment are included in the FAS population.

Safety and tolerability data are summarized descriptively and are not formally analyzed unless otherwise specified; data are presented by treatment group using the Safety analysis set.

Depending on the extent of any impact, summaries of data relating civil crisis, natural disaster, or public health crisis may be generated.

### Calculation or Derivation of Tumor Response Variables

Investigator RECIST 1.1-based assessments: All RECIST 1.1 assessments, whether scheduled or unscheduled, are included in the calculations. This is also regardless of whether a participant discontinues study intervention or receives another anti-cancer therapy.

At each visit, participants are programmatically assigned a RECIST 1.1 visit response of CR, PR, SD, PD, or NE depending on the status of their disease compared with baseline and previous assessments. The tumor response endpoints (PFS, ORR, and DoR) are then derived from the scan dates and overall visit responses.

BICR: A BICR of radiological scans are performed on the PD-L1 high expression population analysis set or the ITT analysis set to confirm the robustness of the investigator-assessed PFS. All images are collected centrally. The imaging scans are reviewed by 2 independent radiologists using RECIST 1.1 and are adjudicated, if required. For each participant, the BICR defines the overall visit response data (CR, PR, SD, PD, or NE) and the relevant scan dates for each time point (*i.e.,* for visits where response or progression is/is not identified). If a participant has had a tumor assessment that cannot be evaluated, then the participant is assigned a visit response of NE (unless there is evidence of progression, in which case the response is assigned as disease progression). The endpoint of PFS is then derived from the scan dates and overall visit responses.

### Primary Endpoint

### Progression-free Survival

Progression-free survival is defined as the time from the date of randomization until tumor progression or death due to any cause regardless of whether the participant withdraws from therapy, receives another anti-cancer therapy or clinically progresses prior to RECIST 1.1 progression. However, if the participant progresses or dies immediately after 2 or more consecutive missed visits, the participant is censored at the time of the last date of assessment which the RECIST 1.1 assessment and response evaluation physical exam were evaluable. The analysis includes all participants with high expression of PD-L1.

The PFS time is derived based on scan/assessment dates and not visit dates.

RECIST 1.1 assessments contributing toward a particular visit may be performed on different dates.
The following rules are applied:
- For investigator assessments, the date of progression is determined based on the earliest RECIST 1.1 assessment/scan dates of the component that indicates progression.
- When censoring a participant for PFS, the participant is censored at the latest scan dates contributing to a particular overall visit assessment.

### Secondary Endpoint(s)

### Progression-free Survival

Progression-free survival is defined as the time from the date of randomization until tumor progression or death due to any cause regardless of whether the participant withdraws from therapy, receives another anti-cancer therapy or clinically progresses prior to RECIST 1.1 progression. However, if the participant progresses or dies immediately after 2 or more consecutive missed visits, the participant is censored at the time of the last date of assessment which the RECIST 1.1 assessment and response evaluation physical exam were evaluable. The analysis includes all participants with high expression of PD-L1 or the ITT analysis set.

The PFS time is based on scan/assessment dates and not visit dates. RECIST 1.1 assessments contributing toward a particular visit may be performed on different dates.

### Overall Survival

Overall survival is defined as time from randomization until the date of death due to any cause. The comparison includes all randomized participants with high expression of PD-L1 or the ITT analysis set, regardless of whether the participants withdraw from the study intervention or receive another anti-cancer therapy.

For assessment of OS at 36 months, Kaplan-Meier plots are presented by treatment group.

Any participant not known to have died at the time of analysis are censored based on the last recorded date on which the participant was known to be alive.

### Objective Response Rate and Complete Response Rate

Objective response rate is defined as the percentage of evaluable patients with an Investigator-assessed visit response of CR or PR. Complete response rate is defined as the disappearance of all target and nontarget lesions. Duration of response is defined as the time from date of first detection of CR until the date of objective disease progression according to RECIST 1.1.

The analysis includes all randomized participants with high expression of PD-L1 or the ITT analysis set with measurable disease at baseline/with evaluable disease at baseline.

Data obtained up until progression, or last evaluable assessment in the absence of progression, is included in the assessment of ORR. Participants who discontinue randomized treatment without progression receive a subsequent anti-cancer therapy and then are included as non-responders in the ORR (note that for this analysis palliative radiotherapy is not considered a subsequent anti-cancer therapy). Participants without an evaluable post baseline assessment are included as non-responders. Participants without a baseline assessment are also included as non-responders unless the participant has a CR at the first post-baseline assessment (unconfirmed response) that is confirmed at the next assessment (confirmed response).

ORR is based on the investigator at local site data and using all scans regardless of whether they were scheduled or not.

### Incidence of Progression or Secondary Malignancy

The incidence of local progression, distant disease progression, and secondary malignancy is defined as the number and percentage of participants who develop local progression, distant disease recurrence, or secondary malignancy.

The comparison includes: all participants in the ITT analysis set. All events are included, regardless of whether the participant withdraws from subsequent therapy and regardless of missed visits.

### Time to First subsequent Therapy (TFST) or Death

The TFST is defined as time from randomization until the start date of the first subsequent anti-cancer therapy after discontinuation of randomized treatment, or death due to any cause. The analysis includes all randomized participants with high expression of PD-L1 or the ITT analysis set.

### Time to Second Progression or Death

Time to second progression or death is defined as the time from randomization to the earliest of the progression event (following the initial investigator-assessed progression), after first subsequent therapy, or death. The date of second progression is recorded by the investigator in the eCRF and defined according to local standard clinical practice.

The analysis includes all randomized participants with high expression of PD-L1 or the ITT analysis set. All events are included, regardless of whether the participant withdraws from subsequent therapy and regardless of missed visits.

Time to second progression or death is analyzed using the same methodology specified for PFS. The effect of volrustomig versus placebo is estimated by the HR together with its corresponding CI and p-value. Kaplan-Meier plots is presented by treatment group.

### Tertiary/Exploratory Endpoint(s)

### Safety

Safety and tolerability is evaluated in terms of AEs, clinical laboratory, vital signs, and ECG. In addition, extra evaluation is done for the following pre-specified AE categories: bleeding, ISRs, thrombocytopenia, neurological events, hypersensitivity; and the pre-specified safety category of new onset or worsening of diabetes. Exposure to study intervention is described.

The aim with the analyses of safety data is to assess the general safety objective, evaluated in a scenario where study intervention is not prematurely discontinued.

### Pharmacokinetics and Pharmacodynamics

Details of PK, population PK, pharmacodynamic, PK/pharmacodynamic relationships, and and/or exposure response/safety analyses are described in the SAP. The population PK analysis and pharmacodynamics analyses are presented separately from the main CSR.

### Biomarkers

Biomarker status is assessed for participants in each treatment group according to pre-specified criteria that may be detailed in the SAP. Biomarker exploratory analyses may be described in a separate analysis plan and may be reported outside the CSR in a separate report. The results of this biomarker assessment is reported either in the CSR itself or as an addendum, or separately in a scientific report or publication. The results of this biomarker assessment may be pooled with biomarker data from other studies with the study intervention to generate hypotheses to be tested in future research.

Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes aspects in which exactly one member of the group is present in, employed in, or otherwise relevant to a given product or process. The disclosure includes aspects in which more than one, or all of the group members are present in, employed in, or otherwise relevant to a given product or process.

Furthermore, the disclosure encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.*, in Markush group format, each subgroup of the elements is also disclosed, and any element can be removed from the group.

It should it be understood that, in general, where the disclosure, or aspects of the disclosure, is/are referred to as comprising particular elements and/or features, certain aspects of the disclosure or aspects of the disclosure consist, or consist essentially of, such elements and/or features. For purposes of simplicity, those aspects have not been specifically set forth *in haec verba* herein.

All patents and publications mentioned in this specification are herein incorporated by reference to the same extent as if each independent patent and publication was specifically and individually indicated to be incorporated by reference. Citation or identification of any reference in any section of this application shall not be construed as an admission that such reference is available as prior art to the present disclosure.

Numbered Embodiments:
1. A method of treating cervical cancer, the method comprising treating the patient with an anti-PD-1 binding protein or an anti-PD-L1 binding protein.
2. The method of embodiment 1, wherein the method further comprises treating the patient with a chemoradiation therapy.
3. The method of either embodiment 1 or embodiment 2, wherein the cervical cancer is locally advanced cervical cancer (LACC).
4. The method of any one of embodiments 1-3, wherein the patient has a tumor area positivity (TAP) score of ≥ 20% for PD-L1 expression.
5. The method of any one of embodiments 1-4, wherein the anti-PD-1 binding protein or the anti-PD-L1 binding protein is an anti-PD-1 antibody selected from pembrolizumab, nivolumab, and cemiplimab.
6. The method of any one of embodiments 1-4, wherein the anti-PD-1 binding protein or the anti-PD-L1 binding protein is an anti-PD-L1 antibody selected from durvalumab, avelumab, atezolizumab, and sugemalimab.
7. The method of any one of embodiments 1-4, wherein the anti-PD-1 binding protein or the anti-PD-L1 binding protein is a bispecific antibody, wherein the bispecific antibody is MEDI5752.
8. The method of any one of embodiments 1-7, wherein the chemoradiation therapy comprises concurrent chemoradiation therapy.
9. The method of any one of embodiments 1-8, wherein the chemoradiation therapy comprises external beam radiotherapy and brachytherapy.
10. The method of any one of embodiments 1-9, wherein the chemoradiation therapy comprises a platinum-based chemotherapy.
11. The method of embodiment 10, wherein the platinum-based chemotherapy is cisplatin or carboplatin.
12. The method of any one of embodiments 5-11, wherein treatment with the anti-PD-L1 antibody comprises administering 1500 mg of the anti-PD-L1 antibody to the patient intravenously every four weeks (Q4W).
13. The method of any one of embodiments 5-11, wherein treatment with the anti-PD-L1 antibody comprises administering 1500 mg of the anti-PD-L1 antibody to the patient intravenously every three weeks (Q3W).
14. The method of either embodiment 12 or embodiment 13, wherein the anti-PD-L1 antibody is durvalumab.
15. The method of any one of embodiments 7-11, wherein treatment with the bispecific antibody comprises administering 750 mg of the bispecific antibody to the patient intravenously every three weeks (Q3W).
16. The method of any one of embodiments 7-11, wherein treatment with the bispecific antibody comprises administering 500 mg or 750 mg of MEDI5752 to the patient intravenously every three weeks (Q3W).
17. The method of any one of embodiments 2-16, wherein the anti-PD-1 binding protein or the anti-PD-L1 binding protein, and the chemoradiation therapy are administered concurrently.
18. The method of any one of embodiments 2-16, wherein the anti-PD-1 binding protein or the anti-PD-L1 binding protein is administered after the chemoradiation therapy.
19. The method of any one of embodiments 1-18, wherein the method extends progression free survival (PFS) in the patient.
20. The method of any one of embodiments 1-18, wherein the method increases the overall response rate (ORR) in the patient.
21. A combination for use in the treatment of cervical cancer in a patient, wherein the combination comprises an anti-PD-1 binding protein or an anti-PD-L1 binding protein.
22. The combination for use of embodiment 21, wherein the combination further comprises a chemoradiation therapy.
23. The combination for use of either embodiment 21 or embodiment 22, wherein the cervical cancer is locally advanced cervical cancer (LACC).
24. The combination for use of any one of embodiments 21-23, wherein the patient has a tumor area positivity (TAP) score of ≥ 20% for PD-L1 expression.
25. The combination for use of any one of embodiments 21-24, wherein the anti-PD-1 binding protein or the anti-PD-L1 binding protein is an anti-PD-1 antibody selected from pembrolizumab, nivolumab, and cemiplimab.
26. The combination for use of any one of embodiments 21-24, wherein the anti-PD-1 binding protein or the anti-PD-L1 binding protein is an anti-PD-L1 antibody selected from durvalumab, avelumab, atezolizumab, and sugemalimab.
27. The combination for use of any one of embodiments 21-24, wherein the anti-PD-1 binding protein is a bispecific antibody, wherein the bispecific antibody is MEDI5752.
28. The combination for use of any one of embodiments 22-27, wherein the chemoradiation therapy comprises concurrent chemoradiation therapy.
29. The combination for use of any one of embodiments 22-28, wherein the chemoradiation therapy comprises external beam radiotherapy and brachytherapy.
30. The combination for use of any one of embodiments 22-28, wherein the chemoradiation therapy comprises a platinum-based chemotherapy.
31. The combination for use of embodiment 30, wherein the platinum-based chemotherapy is cisplatin or carboplatin.
32. The combination for use of any one of embodiments 26 or 28-31, wherein the combination comprises administering 1500 mg of the anti-PD-L1 antibody to the patient intravenously every four weeks (Q4W).
33. The combination for use of any one of embodiments 26 or 28-31, wherein the combination comprises administering 1500 mg of the anti-PD-L1 antibody to the patient intravenously every three weeks (Q3W).
34. The combination for use of either embodiment 32 or embodiment 33, wherein the anti-PD-L1 antibody is durvalumab.
35. The combination for use of any one of embodiments 27-31, wherein the combination treatment comprises administering 500 mg or 750 mg of the bispecific antibody to the patient intravenously every three weeks (Q3W).
36. The combination for use of any one of embodiments 27-31, wherein the combination treatment comprises administering 500 mg or 750 mg of MEDI5752 to the patient intravenously every three weeks (Q3W).
37. The combination for use of any one of embodiments 22-36, wherein the anti-PD-1 binding protein or the anti-PD-L1 binding protein, and the chemoradiation therapy are administered concurrently.
38. The combination for use of any one of embodiments 22-36, wherein the anti-PD-1 binding protein or the anti-PD-L1 binding protein is administered after the chemoradiation therapy.
39. The combination for use of any one of embodiments 21-38, wherein the combination extends progression free survival (PFS) in the patient.
40. The combination for use of any one of embodiments 21-38, wherein the combination increases the overall response rate (ORR) in the patient.
41. Use of an anti-PD-1 binding protein or an anti-PD-L1 binding protein for the manufacture of a medicament for the treatment of cervical cancer in a patient.
42. The use of embodiment 41, wherein the medicament further comprises a chemoradiation therapy.
43. The use of either embodiment 41 or embodiment 42, wherein the cervical cancer is locally advanced cervical cancer (LACC).
44. The use of any one of embodiments 41-43, wherein the patient has a tumor area positivity (TAP) score of ≥ 20% for PD-L1 expression.
45. The use of any one of embodiments 41-44, wherein the anti-PD-1 binding protein or the anti-PD-L1 binding protein is an anti-PD-1 antibody selected from pembrolizumab, nivolumab, and cemiplimab.
46. The use of any one of embodiments 41-44, wherein the anti-PD-1 binding protein or the anti-PD-L1 binding protein is an anti-PD-L1 antibody selected from durvalumab, avelumab, atezolizumab, and sugemalimab.
47. The use of any one of embodiments 41-44, wherein the anti-PD-1 binding protein or the anti-PD-L1 binding protein is a bispecific antibody, wherein the bispecific antibody is MEDI5752.
48. The use of any one of embodiments 42-47, wherein the chemoradiation therapy comprises concurrent chemoradiation therapy.
49. The use of any one of embodiments 42-48, wherein the chemoradiation therapy comprises external beam radiotherapy and brachytherapy.
50. The use of any one of embodiments 42-49 wherein the chemoradiation therapy comprises a platinum-based chemotherapy.
51. The use of embodiment 50, wherein the platinum-based chemotherapy is cisplatin or carboplatin.
52. The use of any one of embodiments 46-51, wherein the treatment comprises administering 1500 mg of the anti-PD-L1 antibody to the patient intravenously every four weeks (Q4W).
53. The use of any one of embodiments 46-51, wherein the treatment comprises administering 1500 mg of the anti-PD-L1 antibody to the patient intravenously every three weeks (Q3W).
54. The use of either embodiment 52 or embodiment 53, wherein the anti-PD-L1 antibody is durvalumab.
55. The use of any one of embodiments 47-51, wherein the treatment comprises administering 500 mg or 750 mg of a bispecific antibody to the patient intravenously every three weeks (Q3W).
56. The use of embodiment 55, wherein the bispecific antibody is MEDI5752.
57. The use of any one of embodiments 42-56, wherein the anti-PD-1 binding protein or the anti-PD-L1 binding protein, and the chemoradiation therapy are administered concurrently.
58. The use of any one of embodiments 42-56, wherein the anti-PD-1 binding protein or the anti-PD-L1 binding protein is administered after the chemoradiation therapy.
59. The use of any one of embodiments 41-58, wherein the treatment extends progression free survival (PFS) in the patient.
60. The use of any one of embodiments 41-58, wherein the treatment increases the overall response rate (ORR) in the patient.
61. A method of treating locally advanced cervical cancer (LACC) in a patient, wherein the method comprises administering 1500 mg of durvalumab to the patient intravenously every four weeks (Q4W), wherein the patient has a tumor area positivity (TAP) score of ≥ 20% for PD-L1 expression.
62. A method of treating locally advanced cervical cancer (LACC) in a patient, wherein the method comprises administering 500 mg or 750 mg of MEDI5752 to the patient intravenously every three weeks (Q3W), wherein the patient has a tumor area positivity (TAP) score of ≥ 20% for PD-L1 expression.
63. The method of either embodiment 61 or embodiment 62, wherein the method further comprises administration of a chemoradiation therapy.
64. The method of embodiment 63, wherein the chemoradiation therapy comprises concurrent chemoradiation therapy.
65. The method of either embodiment 63 or embodiment 64, wherein the chemoradiation therapy comprises external beam radiotherapy and brachytherapy.
66. The method of any one of embodiments 63-65, wherein the chemoradiation therapy comprises a platinum-based chemotherapy.
67. The method of embodiment 66, wherein the platinum-based chemotherapy is cisplatin or carboplatin.
68. The method of any one of embodiments 63-67, wherein durvalumab or MEDI575 and the chemoradiation therapy are administered concurrently.
69. The method of any one of embodiments 63-67, wherein durvalumab or MEDI5752 is administered after the chemoradiation therapy.
70. The method of any one of embodiments 61-69, wherein the method extends progression free survival (PFS) in the patient.
71. The method of any one of embodiments 61-69, wherein the method increases the overall response rate (ORR) in the patient.
72. A combination of durvalumab and a chemoradiation therapy for the treatment of locally advanced cervical cancer (LACC) in a patient, wherein 1500 mg of durvalumab is administered to the patient intravenously every four weeks (Q4W), and wherein the patient has a tumor area positivity (TAP) score of ≥ 20% for PD-L1 expression.
73. A combination of MEDI5752 and a chemoradiation therapy for the treatment of locally advanced cervical cancer (LACC) in a patient, wherein 500 mg or 750 mg of MEDI5752 is administered to the patient intravenously every three weeks (Q3W), and wherein the patient has a tumor area positivity (TAP) score of ≥ 20% for PD-L1 expression.
74. The combination of either embodiment 72 or embodiment 73, wherein the chemoradiation therapy comprises concurrent chemoradiation therapy.
75. The combination of any one of embodiments 72-74, wherein the chemoradiation therapy comprises external beam radiotherapy and brachytherapy.
76. The combination of any one of embodiments 72-75, wherein the chemoradiation therapy comprises a platinum-based chemotherapy.
77. The combination of embodiment 76, wherein the platinum-based chemotherapy is cisplatin or carboplatin.
78. The combination of any one of embodiments 72, 73, or 75-77, wherein durvalumab or MEDI5752 is administered after the chemoradiation therapy.
79. The combination of any one of embodiments 72-78, wherein the combination extends progression free survival (PFS) in the patient.
80. The combination of any one of embodiments 72-78, wherein the combination increases the overall response rate (ORR) in the patient.

## Claims

1. A method of treating cervical cancer, the method comprising treating the patient with an anti-PD-1 binding protein or an anti-PD-L1 binding protein.

2. The method of claim 1, wherein the method further comprises treating the patient with a chemoradiation therapy.

3. The method of either claim 1 or claim 2, wherein the cervical cancer is locally advanced cervical cancer (LACC).

4. The method of any one of claims 1-3, wherein the patient has a tumor area positivity (TAP) score of ≥ 20% for PD-L1 expression.

5. The method of any one of claims 1-4, wherein the anti-PD-1 binding protein or the anti-PD-L1 binding protein is a bispecific antibody, wherein the bispecific antibody is MEDI5752.

6. The method of any one of claims 1-4, wherein the anti-PD-1 binding protein or the anti-PD-L1 binding protein is an anti-PD-L1 antibody selected from durvalumab, avelumab, atezolizumab, and sugemalimab.

7. The method of any one of claims 1-4, wherein the anti-PD-1 binding protein or the anti-PD-L1 binding protein is an anti-PD-1 antibody selected from pembrolizumab, nivolumab, and cemiplimab.

8. The method of any one of claims 1-7, wherein the chemoradiation therapy comprises concurrent chemoradiation therapy.

9. The method of any one of claims 1-8, wherein the chemoradiation therapy comprises external beam radiotherapy and brachytherapy.

10. The method of any one of claims 1-9, wherein the chemoradiation therapy comprises a platinum-based chemotherapy.

11. The method of claim 10, wherein the platinum-based chemotherapy is cisplatin or carboplatin.

12. The method of any one of claims 5-11, wherein treatment with the anti-PD-L1 antibody comprises administering 1500 mg of the anti-PD-L1 antibody to the patient intravenously every four weeks (Q4W).

13. The method of any one of claims 5-11, wherein treatment with the anti-PD-L1 antibody comprises administering 1500 mg of the anti-PD-L1 antibody to the patient intravenously every three weeks (Q3W).

14. The method of either claim 12 or claim 13, wherein the anti-PD-L1 antibody is durvalumab.

15. The method of any one of claims 5 or 8-11, wherein treatment with the bispecific antibody comprises administering 750 mg of the bispecific antibody to the patient intravenously every three weeks (Q3W).

16. The method of any one of claims 5 or8-11, wherein treatment with the bispecific antibody comprises administering 500 mg or 750 mg of MEDI5752 to the patient intravenously every three weeks (Q3W).

17. The method of any one of claims 2-16, wherein the anti-PD-1 binding protein or the anti-PD-L1 binding protein, and the chemoradiation therapy are administered concurrently.

18. The method of any one of claims 2-16, wherein the anti-PD-1 binding protein or the anti-PD-L1 binding protein is administered after the chemoradiation therapy.

19. The method of any one of claims 1-18, wherein the method extends progression free survival (PFS) in the patient.

20. The method of any one of claims 1-18, wherein the method increases the overall response rate (ORR) in the patient.

21. A combination for use in the treatment of cervical cancer in a patient, wherein the combination comprises an anti-PD-1 binding protein or an anti-PD-L1 binding protein.

22. The combination for use of claim 21, wherein the combination further comprises a chemoradiation therapy.

23. The combination for use of either claim 21 or claim 22, wherein the cervical cancer is locally advanced cervical cancer (LACC).

24. The combination for use of any one of claims 21-23, wherein the patient has a tumor area positivity (TAP) score of ≥ 20% for PD-L1 expression.

25. The combination for use of any one of claims 21-24, wherein the anti-PD-1 binding protein is a bispecific antibody, wherein the bispecific antibody is MEDI5752.

26. The combination for use of any one of claims 21-24, wherein the anti-PD-1 binding protein or the anti-PD-L1 binding protein is an anti-PD-L1 antibody selected from durvalumab, avelumab, atezolizumab, and sugemalimab.

27. The combination for use of any one of claims 21-24, wherein the anti-PD-1 binding protein or the anti-PD-L1 binding protein is an anti-PD-1 antibody selected from pembrolizumab, nivolumab, and cemiplimab.

28. The combination for use of any one of claims 22-27, wherein the chemoradiation therapy comprises concurrent chemoradiation therapy.

29. The combination for use of any one of claims 22-28, wherein the chemoradiation therapy comprises external beam radiotherapy and brachytherapy.

30. The combination for use of any one of claims 22-28, wherein the chemoradiation therapy comprises a platinum-based chemotherapy.

31. The combination for use of claim 30, wherein the platinum-based chemotherapy is cisplatin or carboplatin.

32. The combination for use of any one of claims 26 or 28-31, wherein the combination comprises administering 1500 mg of the anti-PD-L1 antibody to the patient intravenously every four weeks (Q4W).

33. The combination for use of any one of claims 26 or 28-31, wherein the combination comprises administering 1500 mg of the anti-PD-L1 antibody to the patient intravenously every three weeks (Q3W).

34. The combination for use of either claim 32 or claim 33, wherein the anti-PD-L1 antibody is durvalumab.

35. The combination for use of any one of claims 25 or 28-31, wherein the combination treatment comprises administering 500 mg or 750 mg of the bispecific antibody to the patient intravenously every three weeks (Q3W).

36. The combination for use of any one of claims 25 or 28-31, wherein the combination treatment comprises administering 500 mg or 750 mg of MEDI5752 to the patient intravenously every three weeks (Q3W).

37. The combination for use of any one of claims 22-36, wherein the anti-PD-1 binding protein or the anti-PD-L1 binding protein, and the chemoradiation therapy are administered concurrently.

38. The combination for use of any one of claims 22-36, wherein the anti-PD-1 binding protein or the anti-PD-L1 binding protein is administered after the chemoradiation therapy.

39. The combination for use of any one of claims 21-38, wherein the combination extends progression free survival (PFS) in the patient.

40. The combination for use of any one of claims 21-38, wherein the combination increases the overall response rate (ORR) in the patient.

41. Use of an anti-PD-1 binding protein or an anti-PD-L1 binding protein for the manufacture of a medicament for the treatment of cervical cancer in a patient.

42. The use of claim 41, wherein the medicament further comprises a chemoradiation therapy.

43. The use of either claim 41 or claim 42, wherein the cervical cancer is locally advanced cervical cancer (LACC).

44. The use of any one of claims 41-43, wherein the patient has a tumor area positivity (TAP) score of ≥ 20% for PD-L1 expression.

45. The use of any one of claims 41-44, wherein the anti-PD-1 binding protein or the anti-PD-L1 binding protein is a bispecific antibody, wherein the bispecific antibody is MEDI5752.

46. The use of any one of claims 41-44, wherein the anti-PD-1 binding protein or the anti-PD-L1 binding protein is an anti-PD-L1 antibody selected from durvalumab, avelumab, atezolizumab, and sugemalimab.

47. The use of any one of claims 41-44, wherein the anti-PD-1 binding protein or the anti-PD-L1 binding protein is an anti-PD-1 antibody selected from pembrolizumab, nivolumab, and cemiplimab.

48. The use of any one of claims 42-47, wherein the chemoradiation therapy comprises concurrent chemoradiation therapy.

49. The use of any one of claims 42-48, wherein the chemoradiation therapy comprises external beam radiotherapy and brachytherapy.

50. The use of any one of claims 42-49 wherein the chemoradiation therapy comprises a platinum-based chemotherapy.

51. The use of claim 50, wherein the platinum-based chemotherapy is cisplatin or carboplatin.

52. The use of any one of claims 46-51, wherein the treatment comprises administering 1500 mg of the anti-PD-L1 antibody to the patient intravenously every four weeks (Q4W).

53. The use of any one of claims 46-51, wherein the treatment comprises administering 1500 mg of the anti-PD-L1 antibody to the patient intravenously every three weeks (Q3W).

54. The use of either claim 52 or claim 53, wherein the anti-PD-L1 antibody is durvalumab.

55. The use of any one of claims 45 or 48-51, wherein the treatment comprises administering 500 mg or 750 mg of a bispecific antibody to the patient intravenously every three weeks (Q3W).

56. The use of claim 55, wherein the bispecific antibody is MEDI5752.

57. The use of any one of claims 42-56, wherein the anti-PD-1 binding protein or the anti-PD-L1 binding protein, and the chemoradiation therapy are administered concurrently.

58. The use of any one of claims 42-56, wherein the anti-PD-1 binding protein or the anti-PD-L1 binding protein is administered after the chemoradiation therapy.

59. The use of any one of claims 41-58, wherein the treatment extends progression free survival (PFS) in the patient.

60. The use of any one of claims 41-58, wherein the treatment increases the overall response rate (ORR) in the patient.

61. A method of treating locally advanced cervical cancer (LACC) in a patient, wherein the method comprises administering 500 mg or 750 mg of MEDI5752 to the patient intravenously every three weeks (Q3W), wherein the patient has a tumor area positivity (TAP) score of ≥ 20% for PD-L1 expression.

62. A method of treating locally advanced cervical cancer (LACC) in a patient, wherein the method comprises administering 1500 mg of durvalumab to the patient intravenously every four weeks (Q4W), wherein the patient has a tumor area positivity (TAP) score of ≥ 20% for PD-L1 expression.

63. The method of either claim 61 or claim 62, wherein the method further comprises administration of a chemoradiation therapy.

64. The method of claim 63, wherein the chemoradiation therapy comprises concurrent chemoradiation therapy.

65. The method of either claim 63 or claim 64, wherein the chemoradiation therapy comprises external beam radiotherapy and brachytherapy.

66. The method of any one of claims 63-65, wherein the chemoradiation therapy comprises a platinum-based chemotherapy.

67. The method of claim 66, wherein the platinum-based chemotherapy is cisplatin or carboplatin.

68. The method of any one of claims 63-67, wherein MEDI5752 or durvalumab and the chemoradiation therapy are administered concurrently.

69. The method of any one of claims 63-67, wherein MEDI5752 or durvalumab is administered after the chemoradiation therapy.

70. The method of any one of claims 61-69, wherein the method extends progression free survival (PFS) in the patient.

71. The method of any one of claims 61-69, wherein the method increases the overall response rate (ORR) in the patient.

72. A combination of MEDI5752 and a chemoradiation therapy for the treatment of locally advanced cervical cancer (LACC) in a patient, wherein 500 mg or 750 mg of MEDI5752 is administered to the patient intravenously every three weeks (Q3W), and wherein the patient has a tumor area positivity (TAP) score of ≥ 20% for PD-L1 expression.

73. A combination of durvalumab and a chemoradiation therapy for the treatment of locally advanced cervical cancer (LACC) in a patient, wherein 1500 mg of durvalumab is administered to the patient intravenously every four weeks (Q4W), and wherein the patient has a tumor area positivity (TAP) score of ≥ 20% for PD-L1 expression.

74. The combination of either claim 72 or claim 73, wherein the chemoradiation therapy comprises concurrent chemoradiation therapy.

75. The combination of any one of claims 72-74, wherein the chemoradiation therapy comprises external beam radiotherapy and brachytherapy.

76. The combination of any one of claims 72-75, wherein the chemoradiation therapy comprises a platinum-based chemotherapy.

77. The combination of claim 76, wherein the platinum-based chemotherapy is cisplatin or carboplatin.

78. The combination of any one of claims 72, 73, or 75-77, wherein MEDI5752 or durvalumab is administered after the chemoradiation therapy.

79. The combination of any one of claims 72-78, wherein the combination extends progression free survival (PFS) in the patient.

80. The combination of any one of claims 72-78, wherein the combination increases the overall response rate (ORR) in the patient.
